(19) Europäisches Patentamt
European Patent Office
Office européen des brevets

(11) EP 1 288 216 A1

(12) **EUROPEAN PATENT APPLICATION**
published in accordance with Art. 158(3) EPC

(43) Date of publication:
**05.03.2003 Bulletin 2003/10**

(21) Application number: **01919884.5**

(22) Date of filing: **10.04.2001**

(51) Int Cl.[7]: **C07D 487/04**, C07D 471/04, C07D 498/04, C07D 513/04, A61K 31/519, A61K 31/55, A61K 31/551, A61K 31/5365, A61K 31/542, A61P 9/10, A61P 25/00, A61P 25/16, A61P 25/28, A61P 25/14, A61P 3/10, A61P 9/06, A61P 19/02, A61P 29/00, A61P 1/04, A61P 35/00

(86) International application number:
**PCT/JP01/03104**

(87) International publication number:
**WO 01/079206 (25.10.2001 Gazette 2001/43)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **18.04.2000 JP 2000116577**

(71) Applicant: **Sumitomo Pharmaceuticals Company, Limited**
**Osaka-shi, Osaka 541-8510 (JP)**

(72) Inventors:
- **MASUMOTO, Shuji**
**Takatsuki-shi, Osaka 569-0857 (JP)**
- **KITANO, Masahumi**
**Takatsuki-shi, Osaka 569-0081 (JP)**
- **OHASHI, Naohito**
**Takatsuki-shi, Osaka 569-1020 (JP)**

(74) Representative: **Cresswell, Thomas Anthony et al**
**J.A. KEMP & CO.**
**14 South Square**
**Gray's Inn**
**London WC1R 5JJ (GB)**

(54) **TRICYCLIC QUINAZOLINEDIONES**

(57) A compound of the formula (1):

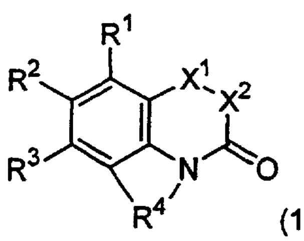

**(1)**

wherein -$X^1$-$X^2$- is a group of the formula: -C(=O)-N($R^7$)- or -C($R^8$)=N- (in which $R^7$ is hydrogen, substituted or unsubstituted alkyl, etc.; and $R^8$ is halogen, etc.); $R^1$, $R^2$ and $R^3$ are independently hydrogen, substituted or unsubstituted alkyl, etc.; and $R^4$ is substituted or unsubstituted alkylene, or a prodrug thereof, or a pharmaceutically acceptable salt of the same, which exhibits an inhibitory activity of poly(ADP-ribose)-polymerase (PARP) and is useful as remedies for diseases caused by the accelerated PARP activity such as brain ischemic disorders.

EP 1 288 216 A1

## Description

### TECHNICAL FIELD

The present invention relates to a compound exhibiting an inhibitory activity of poly(ADP-ribose)polymerase (PARP, also known as poly(ADP-ribose)synthetase). Compounds having a PARP inhibitory activity can be useful as remedies for diseases caused by accelerated PARP activity such as brain ischemic disorders (e.g., stroke, or aftereffects of stroke including disorders accompanied by stroke, disturbances remained as aftereffects of stroke such as movement disorder, brain edema, etc.), neurodegenerative diseases (e.g., Parkinson's disease, Alzheimer's disease, Huntington's chorea, etc.), brain contusion, head injury, spinal injury, diabetes mellitus, ischemic heart disease (e.g., myocardial infarction, angina pectris, arrhythmia, etc.), organ damages caused by ischemia or ischemic reperfusion injury (e.g., myocardial ischemic reperfusion injury, acute renal failure, renal ischemia, injuries caused by surgical operations such as organ transplant or percutaneous transluminal coronary angioplasty, etc.), inflammations (e.g., arthritis, rheumatoid arthritis, septic shock), inflammatory enteritis (e.g., colitis, Crohn's disease, etc.), cancers, cachexy, renal damage, osteoporosis, acute pain and chronic pain (e.g., neurogenic pain, etc.), septic shock (e.g., endotoxin shock, etc.), skeletal muscle degenerative disease, muscular dystrophy, skin aging, aging of immune system, AIDS, alteration of gene expression of senescent cells, etc.

Especially, the present compound is useful as remedies for brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, skin aging, etc.

### BACKGROUND ART

As a compound having an inhibitory activity of poly(ADP-ribose)polymerase, there have been known dihydroisoquinolinone derivatives and isoquinolinone derivatives as disclosed in Anti-cancer Drug Design (1991), 7, 107-117, bisbenzamide derivatives as disclosed in WO 99/47494, tetracyclic compounds as disclosed in WO 99/11645, etc., and J. Biol. Chem. (1992), 267 (3), 1569-1575 also discloses compounds of various nucleuses having poly(ADP-ribose) polymerase inhibitory activity.

### DISCLOSURE OF INVENTION

Recently, although PARP inhibitors having various chemical structures as mentioned above have been found, it has been desired to develop a compound having a more potent PARP inhibitory activity with few side effects.

The present inventors have intensively studied in order to solve the above-mentioned problems, and have found that a compound of the following formula (1), or a prodrug thereof, or a pharmaceutically acceptable salt of the same (hereinafter, optionally referred to as "the present compound") exhibits an excellent poly(ADP-ribose)polymerase inhibitory activity, and have accomplished the present invention. That is, the present invention relates to the following embodiments:

[1] A compound of the formula (1):

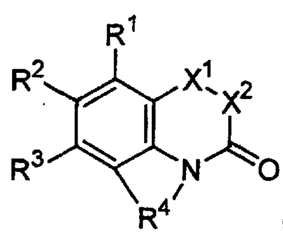

(1)

wherein $-X^1-X^2-$ is a group of the formula: $-C(=O)-N(R^7)-$ or $-C(R^8)=N-$ (in which $R^7$ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated, heterocyclic group, or a substituted or unsubstituted acyl group, $R^8$ is a halogen atom or a group of the formula: $-OR^{8a}$, $-NH_2$, $-NHR^{8a}$, $-NR^{8a}R^{8b}$ or $-SR^{8a}$ ($R^{8a}$ and $R^{8b}$ are independently a substituted or unsubstituted alkyl group));

$R^1$, $R^2$ and $R^3$ are independently a hydrogen atom, a substituted or unsubstituted alkyl group, a substituted or

unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated heterocyclic group, a substituted or unsubstituted acyl group, a halogen atom, a nitro group, or a group of the formula: $OR^{1a}$ $-NR^{1a}R^{1b}$ or $-SR^{1a}$ (in which $R^{1a}$ and $R^{1b}$ are independently a hydrogen atom, or a substituted or unsubstituted alkyl group);

$R^4$ is a substituted or unsubstituted alkylene group (in which the $-CH_2-$ moiety of said alkylene group may optionally be replaced by one or more groups which are the same or different, selected from the group consisting of -O-, $-S(O)_n-$, $-N(R^{6a})-$, $-C(=N-OR^{6b})-$, $-C(=CR^{6c}R^{6d})-$, and -C(=O)-, and any combination of two adjacent carbon atoms of said alkylene group may optionally form a double bond or a triple bond, n is an integer of 0, 1 or 2, $R^{6a}$ is a substituted or unsubstituted alkyl group, a substituted or unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated heterocyclic group, or a substituted or unsubstituted acyl group, $R^{6b}$ is a hydrogen atom, a substituted or unsubstituted alkyl group, or a substituted or unsubstituted arylalkyl group, $R^{6c}$ and $R^{6d}$ are independently a hydrogen atom or a substituted or unsubstituted lower alkyl group),

provided that 1H,5H-pyrido[3,2,1-ij]quinazoline-1,3,7(2H,6H)-trione, 9-methyl-5,6-diphenyl- 1H-pyrrolo[3,2,1-ij]quinazoline-1,3(2H)-dione, and 9-methoxy-5,6-diphenyl-1H-pyrrolo[3,2,1-ij]quinazoline-1,3(2H)-dione are excluded, or a prodrug thereof, or a pharmaceutically acceptable salt of the same.

[2] The compound according to the above [1], or a prodrug thereof, or a pharmaceutically acceptable salt of the same, wherein $R^4$ is a substituted or unsubstituted $C_{2-5}$ alkylene group (in which the $-CH_2-$moiety of said alkylene group may optionally be replaced by one or more groups which are the same or different, selected from the group consisting of -O-, $-S(O)_n-$, $-N(R^{6a})-$, $-C(=N-OR^{6b})-$, $C(=CR^{6c}R^{6d}$, and -C(=O)-, and any combination of two adjacent carbon atoms of said alkylene group may optionally form a double bond or a triple bond, and n, $R^{6a}$, $R^{6b}$, $R^{6c}$, and $R^{6d}$ are as defined in the above [1]).

[3] The compound according to the above [1], or a prodrug thereof, or a pharmaceutically acceptable salt of the same, wherein $R^4$ is a substituted or unsubstituted $C_{2-5}$ alkylene group (in which the $-CH_2-$moiety of said alkylene group may optionally be replaced by one or more groups which are the same or different, selected from the group consisting of $-C(=N-OR^{6b})-$, $-C(=CR^{6C}R^{6d})-$, and -C(=O)-, and any combination of two adjacent carbon atoms of said alkylene group may optionally form a double bond or a triple bond, and $R^{6b}$, $R^{6c}$, and $R^{6d}$ are as defined in the above [1]).

[4] The compound according to the above [1], or a prodrug thereof, or a pharmaceutically acceptable salt of the same, wherein $R^4$ is a substituted or unsubstituted $C_{2-5}$ alkylene group.

[5] The compound according to any one of the above [1] to [4], or a prodrug thereof, or a pharmaceutically acceptable salt of the same, wherein $R^4$ has at least one substituent, and at least one of said substituents is a substituted alkyl group of the formula: $-R^{4a}-R^{4b}-R^{4c}-R^{4d}$ (in which $R^{4a}$ is a substituted or unsubstituted alkylene group (among $-CH_2-$groups of said alkylene group, one $-CH_2-$ group other than one directly binding to $R^4$ may optionally be replaced by an oxygen atom or a group of the formula: $-NR^{4e}C(=O)-$ or $-C(=O)NR^{4e}-$ ($R^{4e}$ is a hydrogen atom, a lower alkyl group, or an arylalkyl group)), $R^{4b}$ is a substituted or unsubstituted aromatic group, a cycloalkyl group, or a single bond, $R^{4c}$ is a substituted or unsubstituted alkylene group (one of the $-CH_2-$groups of said alkylene group may optionally be replaced by an oxygen atom) or a single bond, $R^{4d}$ is a hydrogen atom, an amino group or a saturated heterocyclic group containing a nitrogen atom (said amino group or the nitrogen atom of the saturated heterocyclic group containing a nitrogen atom may optionally have one or two lower alkyl substitutents or arylalkyl substitutents, which are the same or different)).

[6] A medicament, which comprises the compound as set forth in any one of the above [1] to [5], or a prodrug thereof, or a pharmaceutically acceptable salt of the same.

[7] A poly(ADP-ribose)polymerase inhibitor, which comprises the compound as set forth in any one of the above [1] to [5], or a prodrug thereof, or a pharmaceutically acceptable salt of the same.

[8] An agent for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, or skin aging, which comprises the compound as set forth in any one of the above [1] to [5], or a prodrug thereof, or a pharmaceutically acceptable salt of the same.

[9] A use of the compound as set forth in any one of the above [1] to [5], or a prodrug thereof, or a pharmaceutically acceptable salt of the same, in preparation of a poly(ADP-ribose)polymerase inhibitor.

[10] A use of the compound as set forth in any one of the above [1] to [5], or a prodrug thereof, or a pharmaceutically acceptable salt of the same, in preparation of an agent for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction,

myocardial ischemic reperfusion injury, angina pectris, arrhythmia, arthritis, rheumatoid arthritis, inflammatory enteritis, septic shock, cancers, or skin aging.

[11] A method for inhibiting poly(ADP-ribose)polymerase in a patient in need, which comprises administering to said patient the compound as set forth in any one of the above [1] to [5], or a prodrug thereof, or a pharmaceutically acceptable salt of the same.

[12] A method for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, or skin aging, which comprises administering to a patient in need the compound as set forth in any one of the above [1] to [5], or a prodrug thereof, or a pharmaceutically acceptable salt of the same.

In the present specification, the compound of the formula (1), or a prodrug thereof, or a pharmaceutically acceptable salt of the same is optionally abbreviated to "the present compound(s)".

Each group in the present invention is explained below. Unless defined otherwise, the following definition for each group should be applied to cases wherein said group is a part of another substituent.

The alkyl group includes, for example, a straight chain or branched chain alkyl group having carbon atoms of not more than 8, such as methyl, ethyl, propyl, 2-propyl, butyl, 2-butyl, 2-methylpropyl, 1,1-dimethylethyl, pentyl, hexyl, heptyl, octyl, etc.

The alkenyl group includes, for example, a straight chain or branched chain alkenyl group having carbon atoms of not more than 6, such as vinyl, allyl, propenyl, 2-propenyl, butenyl, pentenyl, hexenyl, etc.

The alkynyl group includes, for example, an alkynyl group having carbon atoms of not more than 6, such as ethynyl, propargyl, butynyl, pentynyl, etc.

The cycloalkyl group includes, for example, a 3- to 8-membered cycloalkyl group such as cyclopropyl, cyclobutyl, cyclopentyl, cyclohexyl, cycloheptyl, etc.

The cycloalkenyl group includes, for example, a 3- to 8-membered cycloalkenyl group having one double bond, such as 1-cyclopentenyl, 2-cyclopentenyl, 3-cyclopentenyl, 1-cyclohexenyl, 2-cyclohexenyl, 3-cyclohexenyl, etc.

The cycloalkylalkyl group includes, for example, the above alkyl group being substituted by the above cycloalkyl group.

The aromatic group includes, for example, an aryl group and a heteroaryl group.

The aryl group includes, for example, an aryl group having carbon atoms of not more than 10, such as phenyl group, naphthyl group, etc.

The heteroaryl group includes, for example, a 5- or 6-membered monocyclic group having 1 to 2 nitrogen atoms, a 5- or 6-membered monocyclic group having 1 to 2 nitrogen atoms and one oxygen atom or one sulfur atom, a 5-membered monocyclic group having one oxygen atom or one sulfur atom, a bicyclic group formed by fusing a 6-membered ring and a 5- or 6-membered ring and having 1 to 4 nitrogen atoms, such as 2-pyridyl, 3-pyridyl, 4-pyridyl, 2-thienyl, 3-thienyl, 3-oxadiazolyl, 2-imidazolyl, 2-thiazolyl, 3-isothiazolyl, 2-oxazolyl, 3-isoxazolyl, 2-furyl, 3-furyl, 3-pyrrolyl, 2-quinolyl, 8-quinolyl, 2-quinazolinyl, 8-purinyl, etc.

The halogen atom is iodine atom, fluorine atom, chlorine atom, or bromine atom.

The arylalkyl group includes, for example, the above alkyl group being substituted by the above aryl group.

The saturated heterocyclic group includes, for example, a 5- to 8-membered cyclic group having one nitrogen atom such as 1-piperidinyl, 1-pyrrolidinyl, etc., a 6- to 8-membered cyclic group having two nitrogen atoms such as 1-piperazinyl group, a 6- to 8-membered cyclic group having one nitrogen atom and one oxygen atom such as morpholino group.

The saturated heterocyclic group having nitrogen atoms includes, for example, a 5- to 8-membered cyclic group having one nitrogen atom such as 1-piperidinyl group, 1-pyrrolidinyl group, etc., a 6- to 8-membered cyclic group having two nitrogen atoms such as 1-piperazinyl group, a 6- to 8-membered cyclic group having one nitrogen atom and one oxygen atom such as morpholino group.

The substituents of the saturated heterocyclic group, the saturated heterocyclic group having nitrogen atoms, and the saturated heterocyclic group-substituted carbonyl group are, for example, a hydroxy group, a carboxyl group, a halogen atom, an alkoxycarbonyl group, etc. as a substituent on the carbon atom, and an alkyl group, an arylalkyl group, an alkoxycarbonyl group, etc. as a substituent on the nitrogen atom.

The acyl group includes, for example, a formyl group, an alkanoyl group having 2 to 6 carbon atoms such as acetyl, propanoyl, etc., a cycloalkanecarbonyl group having 4 to 7 carbon atoms such as cyclopropanecarbonyl, cyclobutanecarbonyl, cyclopentanecarbonyl, cyclohexanecarbonyl, etc., a cycloalkenecarbonyl group having 4 to 7 carbon atoms such as cyclopentenecarbonyl, cyclohexenecarbonyl, etc., an aroyl group having 7 to 11 carbon atoms such as benzoyl, toluoyl, naphthoyl, etc., a saturated heterocyclic group-substituted carbonyl group wherein the saturated heterocyclic group is a 5- or 6-membered saturated heterocyclic group containing 1 to 2 hetero atoms selected from a nitrogen atom, an oxygen atom and a sulfur atom, such as 2-piperidinecarbonyl, 3-morpholinecarbonyl, etc., a heteroaromatic acyl group having a 5- or 6-membered aromatic heterocyclic group containing 1 to 2 hetero atoms selected from a nitrogen atom, an oxygen atom and a sulfur atom such as 2-furoyl, 3-furoyl, 2-thenoyl, 3-thenoyl, nicotinoyl, isonicotinoyl, etc.

The substituted alkyl group, the substituted alkenyl group, the substituted alkynyl group, the substituted cycloalkyl group, the substituted cycloalkylalkyl group, the substituted alkanoyl group, the substituted cycloalkanecarbonyl group and the substituted cycloalkenecarbonyl group, and the alkyl moiety of the substituted arylalkyl group have one or more substituents, which are the same or different, and the substituents are, for example, a halogen atom, a cyano group, a phenoxy group, a benzyloxy group, a trifluoromethyl group, a hydroxy group, a lower alkoxy group, a lower alkanoyloxy group, an amino group, a mono-lower alkylamino group, a di-lower alkylamino group, a lower alkyl (arylalkyl)amino group, a carbamoyl group, a lower alkylaminocarbonyl group, a di-lower alkylaminocarbonyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkanoylamino group being substituted by an amino group, an aroylamino group, an amino group being substituted by a heteroaromatic acyl group, a lower alkylsulfonamido group, a phthalimido group, a heteroaryl group, or a saturated heterocyclic group.

The substituted aromatic group, the substituted aroyl group and the substituted heteroaromatic acyl group, and the alkyl moiety of the substituted arylalkyl group have one or more substituents, which are the same or different, and the substituents are, for example, a halogen atom, a cyano group, a trifluoromethyl group, a nitro group, a hydroxy group, a methylenendioxy group, a lower alkyl group, a lower alkoxy group, a benzyloxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, or a lower alkylsulfonamido group.

The term "lower" in the present invention means that an alkyl moiety described with "lower" is a lower alkyl group, and the lower alkyl group includes one having carbon atoms of not more than 4 such as methyl, ethyl, propyl, isopropyl, butyl, isobutyl, sec-butyl, t-butyl, etc.

The alkylene group includes, for example, an alkylene group having carbon atoms of not more than 10, preferably having 2 to 5 carbon atoms, such as methylene, ethylene, trimethylene, tetramethylene, pentamethylene, hexamethylene, etc.

The substituted alkylene group has one or more substituents, which are the same or different, and the substituents are, for example, an alkyl group, a substituted alkyl group, a hydroxy group, a halogen atom, a cycloalkyl group, a cycloalkenyl group, a formyl group, a carboxyl group, an alkoxycarbonyl group, a saturated heterocyclic group, an amino group (wherein said amino group may have one or two lower alkyl substituents or arylalkyl substituents on the nitrogen atom thereof, which are the same or different), and an aromatic group. The substituent of $R^4$ is preferably a substituted alkyl group, especially a substituted alkyl group of the formula: $-R^{4a}-R^{4b}-R^{4c}-R^{4d}$ (in which $R^{4a}$, $R^{4b}$, $R^{4c}$ and $R^{4d}$ are as defined above), and especially preferable one is a substituted alkyl group of the formula: $-R^{4a}-R^{4b}-R^{4c}-R^{4d}$ wherein at least one of $-CH_2-$ groups of the alkylene group for $R^4a$ is replaced by at least one group of the formula: $-NR^{4e}C(=O)-$ or $-C(=O)NR^{4e}-$.

The preferable substituent of the lower alkyl group for $R^{6c}$ and $R^{6d}$ is, for example, a halogen atom or a lower alkoxy group.

The compound of the formula (1) may be prepared from publicly known compounds by a combination of the publicly known methods, for example, by the following method.

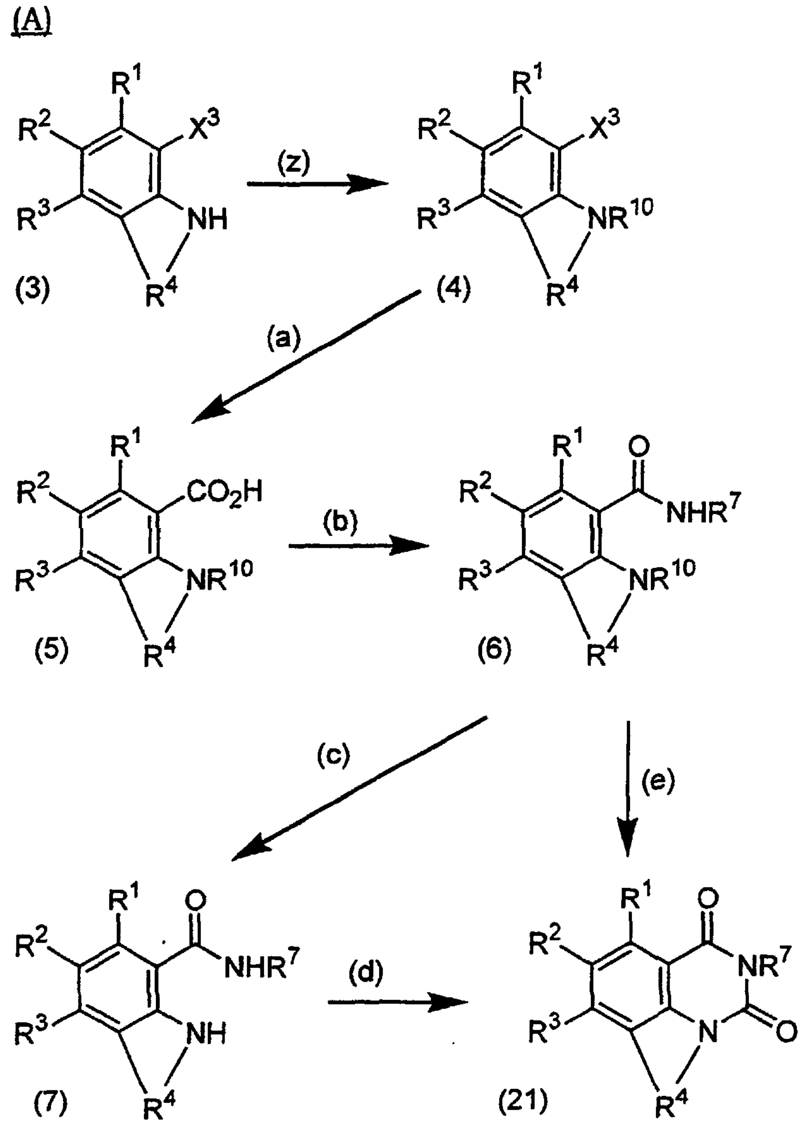

wherein $R^1$, $R^2$, $R^3$, $R^4$ and $R^7$ are as defined above in the above [7], $X^3$ is a hydrogen atom or a halogen atom, and $R^{10}$ is a protecting group for amino group such as an alkoxycarbonyl group.

The starting compound of the formula (3) may be a publicly known compound or can be prepared from a publicly known compound by a combination of the conventional methods. The conventional methods are as follows.

(i) A method wherein a quinoline derivative is subjected to reduction by using a catalyst (e.g., platinum oxide, etc.) in a solvent (e.g., methanol, etc.) at a temperature of from 0°C to a boiling point of the solvent to be used under pressure, if necessary, to give a corresponding tetrahydroquinoline derivative (cf., the method disclosed in Synth. Commun. (1990), 20(22), 3553-3562, etc.).

(ii) A method wherein a cyclic amide derivative is subjected to reduction by using a reducing agent (e.g., aluminum lithium hydride, borane, etc.) in a solvent (e.g., tetrahydrofuran, etc.) at a temperature of 0°C to a boiling point of the solvent to be used to give a corresponding cyclic amine derivative (cf., the method disclosed in Chem. Pharm. Bull. (1996), 44(1), 103-114, J. Org. Chem. (1950), 15, 517, etc.).

(iii) A method of cyclization reaction as shown in the following formula:

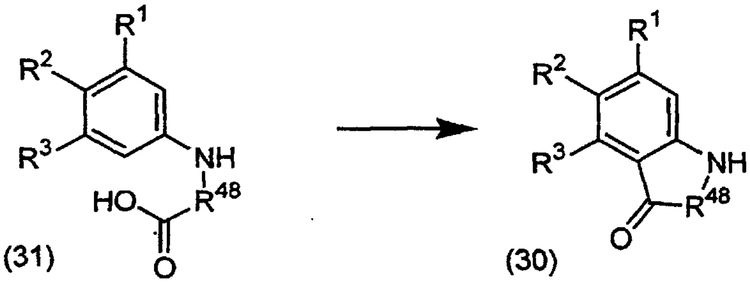

(31) (30)

wherein $R^1$, $R^2$ and $R^3$ are as defined in the above [1], $R^{48}$ is a substituted or unsubstituted alkylene group (in which the $-CH_2-$ moiety of said alkylene group may optionally be replaced by one or more groups which are the same or different, selected from the group consisting of -O-, $-S(O)_n-$, $-N(R^{6a})-$, $-C(=N-OR^{6b})-$, $-C(CR^{6c}R^{6d})-$, and -C(=O)-, and any combination of two adjacent carbon atoms of said alkylene group may optionally form a double bond or a triple bond, and n, $R^{6a}$, $R^{6b}$, $R^{6c}$ and $R^{6d}$ are as defined in the above [1].

The carboxylalkylaniline derivative of the formula (31) is converted into a compound of the formula (30) by a cyclization method disclosed in Tetrahedron Asymmetry (1998), 9(7), 1137-1142, for example, in polyphosphoric acid at a temperature of from 100°C to 200°C, or by a modified method thereof, for example, a method of cyclization reaction in a polyphosphoric acid ester.

The step (z) is a step of protecting the amino group of the compound (3) by $R^{10}$ to give a compound of the formula (4), and when the protecting group is a t-butoxycarbonyl group, then this step (z) is carried out by using di-tert-butyl dicarbonate in a solvent such as aromatic hydrocarbon solvents (e.g., benzene, toluene, xylene, etc.), ether solvents (e.g., tetrahydrofuran, 1,4-dioxane, etc.), halogenated hydrocarbon solvents (e.g., dichloromethane, chloroform, 1,2-dichloro-ethane, etc.), ester solvents (e.g., ethyl acetate, etc.), or a mixture of these solvents, at a temperature of from 0°C to a boiling point of the solvent to be used. Alternatively, as a protecting group for amine group, various protecting groups such as ones as disclosed in Protective Groups in Organic Synthesis, JOHN WILLEY & SONS, 1991 may be used.

The step (a) is a step of introducing a carboxylic acid at the ortho-position of the nitrogen atom to give a compound of the formula (5), which is carried out, for example, by reacting the compound (4) with an organic lithium reagent in an ether solvent such as tetrahydrofuran, diethyl ether, etc., at a temperature of from -100°C to 0°C, followed by reacting the resultant with carbon dioxide (i.e., the same method as disclosed in Heterocycles (1992), 34(5), 1031-1038 with respect to the compound (4) wherein $X^3$ is a hydrogen atom).

The step (b) is a step of amidating the carboxylic acid compound (5) to give a compound of the formula (6), which is carried out, for example, by reacting the carboxylic acid compound (5) with an amine of the formula: $H_2NR^7$ in the presence of a condensing agent in an inert solvent at room temperature or under heating. The reaction is preferably carried out, for example, in the presence of a condensing agent such as dicyclohexylcarbodiimide (DCC), diisopropylcarbodiimide (DIPC), 1-ethyl-3-(3-dimethylaminopropyl)carbodiimide (WSC), benzotriazol-1-yl-tris(dimethylamino)phosphonium hexafluorophosphide (BOP), diphenylphosphonyl azide (DPPA), N,N-carbonyldiimidazole (Angew. Chem. Int. Ed. Engl., Vol. 1, 351 (1962)), etc., and if necessary, in the presence of an additive such as N-hydroxysuccinimde (HONSu), 1-hydroxybenzotriazole (HOBt), 3-hydroxy-4-oxo-3,4-dihydro-1,2,3-benzotriazine (HOObt), etc., in aromatic hydrocarbon solvents (e.g., benzene, toluene, xylene, etc.), ether solvents (e.g., tetrahydrofuran, 1,4-dioxane, etc.), halogenated hydrocarbon solvents (e.g., dichloromethane, chloroform, 1,2-dichloroethane, etc.), amide solvents (e.g., dimethylformamide, dimethylacetamide, etc.), basic solvents (e.g., pyridine, etc.), or a mixture of these solvents.

Alternatively, there is also known a method wherein the carboxylic acid compound (5) is converted into a reactive derivative thereof as an intermediate, which is further reacted with the amine compound. The reactive derivative of the carboxylic acid compound (5) may be, for example, an acid halide, an acid anhydride (including a mixed acid anhydride), or ester derivative thereof. The acid halide is, for example, an acid chloride and an acid bromide, and the mixed acid anhydride is, for example, a mixed acid anhydride with an alkyloxycarbonyl chloride such as ethyloxycarbonyl chloride, isobutyloxycarbonyl chloride, etc., or with an α-polyalkyl-substituted carboxylic acid chloride compound such as 2-ethyl-n-butyryl chloride, trimethylacetyl chloride, and the ester derivative is, for example, an active ester such as p-nitrophenyl ester, N-hydroxysuccinimide ester, pentafluorophenyl ester, etc. or a conventional ester compound such as methyl ester, ethyl ester, etc. These reactive derivatives of a carboxylic acid compound can easily be obtained from a corresponding carboxylic acid by a conventional method that is usually employed.

When reacting with an acid halide or an acid anhydride (including a mixed acid anhydride), the reaction is carried out in the presence of a bas or an excess amount of amine in a solvent under cooling or at room temperature.

The base may be an inorganic base such as sodium hydroxide, potassium hydroxide, sodium carbonate, potassium carbonate, sodium hydrogen carbonate, etc., and an organic base such as triethylamine, pyridine, etc., and the solvent may be aromatic hydrocarbon solvents (e.g., benzene, toluene, xylene, etc.), ether solvents (e.g., tetrahydrofuran, 1,4-dioxane, etc.), halogenated hydrocarbon solvents (e.g., dichloromethane, chloroform, 1,2-dichloro-ethane, etc.), amide solvents (e.g., dimethylformamide, dimethylacetamide, etc.), basic solvents (e.g., pyridine, etc.), or a mixture of these solvents.

When reacting with an ester derivative, the reaction is carried out in the presence of equimolar amount or excess amount of an amine compound in a solvent under cooling or with heating. In the case of an active ester, the reaction is carried out, for example, in a solvent such as an ether solvent (e.g., tetrahydrofuran, 1,2-dimethoxyethane, dioxane, etc.), an ester solvent (e.g., ethyl acetate, etc.), dimethylformamide, or a mixture of these solvents. In the case of other esters, the reaction is carried out in a solvent such as an alcohol solvent (e.g., methanol, ethanol, isopropanol, etc.), an ether solvent (e.g., tetrahydrofuran, 1,2-dimethoxyethane, dioxane, etc.), dimethylformamide, or a mixture of these solvents. In some cases, after the solvent is evaporated off, it may be possible to heat the reaction system at around 130°C for a short period of time.

The step (c) is a step of removing a protecting group $R^{10}$ to give a compound of the formula (7). When the protecting group is t-butoxycarbonyl, the reaction is carried out, for example, by a method of using hydrogen chloride in dioxane, or by a method of using hydrochloric acid in acetic acid at a temperature of from room temperature to a boiling point of the solvent. The removal of other various protecting groups for amine may be carried out, for example, by a method disclosed in Protective Groups in Organic Synthesis, JOHN WILLEY & SONS, 1991.

In addition, there may be generated an acidic hydrogen halide in preparation of a reactive derivative of the carboxylic acid compound in the above step (b), especially in preparation of an acid halide compound thereof, and in those cases, the step (c) of removing a protecting group $R^{10}$ is not necessarily needed depending on the kinds of said protecting group to be removed.

The step (d) is a step of cyclizing the compound (7) to give a compound of the formula (21). The reaction is carried out, for example, by using a reagent such as N,N-carbonyldiimidazole, a haloformic acid ester, triphosgene, etc. in a suitable solvent, or by using such a reagent as a solvent, at a temperature of from room temperature to a boiling point of the solvent. When a solvent is used, it is, for example, aromatic hydrocarbon solvents (e.g., benzene, toluene, xylene, etc.), ether solvents (e.g., tetrahydrofuran, 1,4-dioxane, etc.), halogenated hydrocarbon solvents (e.g., dichloromethane, chloroform, 1,2-dichloroethane, etc.), amide solvents (e.g., dimethylformamide, dimethylacetamide, etc.), basic solvents (e.g., pyridine, etc.), or a mixture of these solvents.

When $R^{10}$ is an alkoxycarbonyl group, etc., it may be possible to directly obtain the compound of the formula (21) by cyclization reaction of the step (e). The cyclization reaction is preferably carried out in the presence of a base (e.g., sodium hydroxide, potassium t-butoxide, etc.), for example, in an aromatic hydrocarbon solvent (e.g., benzene, toluene, xylene, etc.), an amide solvent (e.g., dimethylformamide, dimethylacetamide, etc.), a basic solvent (e.g., pyridine, etc.), or a mixture of these solvents, at a temperature of from 0°C to a boiling point of the solvent.

(B)

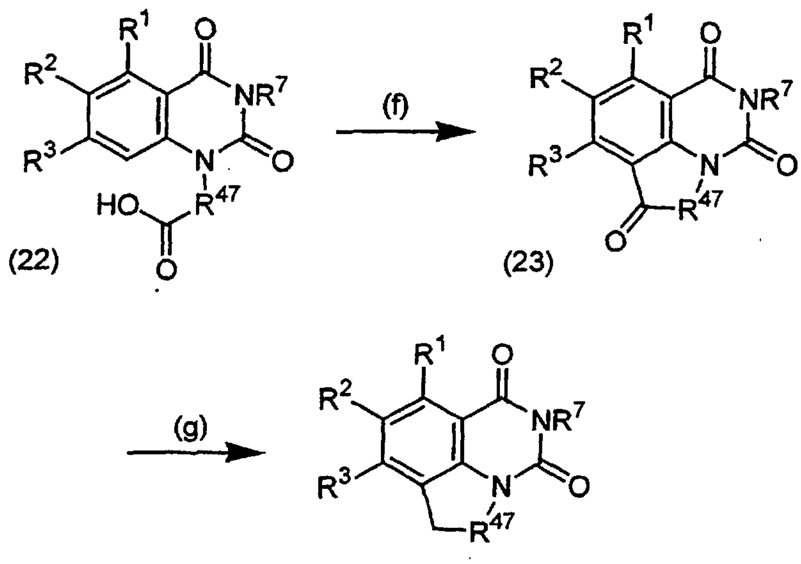


(22) (23)

(24)

wherein $R^1$, $R^2$, $R^3$ and $R^7$ are as defined in the above [1], $R^{47}$ is a substituted or unsubstituted alkylene group (in which the $-CH_2-$ moiety of said alkylene group may optionally be replaced by one or more groups which are the same or different, selected from the group consisting of -O-, $-S(O)_n-$, $-N(R^{6a})-$, $-C(=N-OR^{6b})-$, $-C(CR^{6c}R^{6d})-$, and -C(=O)-, and any combination of two adjacent carbon atoms of said alkylene group may optionally form a double bond or a triple bond, and n, $R^{6a}$, $R^{6b}$, $R^{6c}$ and $R^{6d}$ are as defined in the above [1]).

The step (f) is a step of cyclizing the carboxylic acid compound (22) to give a compound of the formula (23). The cyclization reaction is carried out in polyphosphoric acid or a polyphosphoric acid ester, for example, by a modified method of the method of using a mixture of phosphorus pentachloride and phosphoric acid in a ratio of 2.5:1 and heating the mixture at 140°C as disclosed in Chem. Heterocycl. Compd. (1997), 33(1), 96-98.

Alternatively, the step (f) may be carried out by a conventional Friedel-Crafts reaction. For example, the carboxylic acid compound (22) is treated with thionyl chloride or phosphorus pentachloride to give an acid halide, which is subjected to cyclization reaction by using a Lewis acid such as aluminum chloride, antimony pentachloride, iron trichloride, tin tetrachloride, titanium tetrachloride, zinc chloride, boron trifluoride, etc. The solvent used in this reaction is, for example, nitrobenzene, 1,2-dichloroethane, chloroform, acetone, tetrahydrofuran, ethyl acetate, etc.

The step (g) is a step of reducing the compound (23) to give a compound of the formula (24), and it is carried out by a conventional reduction for a ketone at benzyl position, for example, by using triethyl silane in a trifluoroacetic acid.

In addition, by selecting the reduction conditions, there may be obtained a compound of the formula (24) wherein the carbonyl group is converted into hydroxymethylene group, and said hydroxymethylene group can be converted into other substituents by a conventional method.

(C)

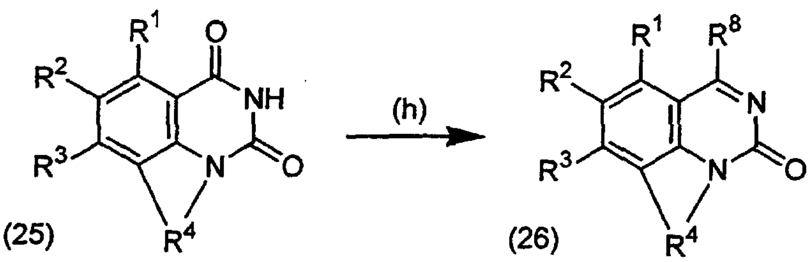


(25) (26)

wherein $R^1$, $R^2$, $R^3$, $R^4$ and $R^8$ are as defined in the above [1].

The step (h) is a step of obtaining a compound of the formula (26) from the compound (25), i.e., the compound (21) wherein $R^7$ is a hydrogen atom. When $R^8$ is $NH_2$, the reaction is carried out by heating the compound (25) under reflux in the presence of N,N-diethylaniline in phosphorus oxychloride, followed by reacting the resulting intermediate in a saturated solution of ammonia in methanol at a temperature of from room temperature or under reflux, preferably at 60°C (cf., Tetrahedron Letters (1994), 35(3), 397-400). When $R^8$ is a group of the formula: $-OR^{8a}$, $-NH_2$, $-NHR^{8a}$, $-NR^{8a}R^{8b}$ or $-SR^{8a}$, the reaction may be carried out likewise.

The compound (26) wherein $R^8$ is a halogen atom can be obtained by reacting the compound (25) with a phosphorus oxyhalide (e.g., phosphorus oxychloride, etc.) or a phosphorus halide (e.g., phosphorus pentachloride, phosphorus tribromide, etc.).

In each of the above steps, when the starting compound of each step has a group being active to each reaction such as a hydroxy group, an amino group or a carboxyl group, then such active groups other than groups to be reacted in each reaction are previously protected by a suitable protecting group, and after the reaction is completed or some steps of the reaction are completed, then these protecting groups may be removed to give a desired compound. The protecting groups for hydroxy group, amino group, carboxyl group, etc., may be conventional protecting groups which are used in the field of the organic chemistry, and these protecting groups may be introduced and removed by a conventional method, such as by a method disclosed in PROTECTIVE GROUPS IN ORGANIC SYNTHESIS, John Wiley & Sons, Inc.; 1991.

For example, the protecting group for hydroxy group may be methoxymethyl group, tetrahydropyranyl group, etc., and the protecting group for amino group may be t-butyloxycarbonyl group, etc. These protecting groups for hydroxy group may be removed, for example, by reacting in the presence of an acid such as hydrochloric acid, sulfuric acid, acetic acid, etc., in a solvent such as aqueous methanol, aqueous ethanol, aqueous tetrahydrofuran, etc. The protecting groups for amino group may be removed, for example, by reacting in the presence of an acid such as hydrochloric acid, trifluoroacetic acid, etc. in a solvent such as aqueous tetrahydrofuran, methylene chloride, chloroform, aqueous methanol, etc.

When a carboxyl group is protected, it is protected, for example, by tert-butyl ester, ortho-ester, an acid amide, etc. When the protecting group is tert-butyl ester, the removal thereof is carried out by reacting in the presence of hydrochloric acid in an aqueous solvent. When the protecting group is ortho-ester, the removal thereof is carried out by treating with an acid in a solvent such as aqueous methanol, aqueous tetrahydrofuran, aqueous 1,2-dimethoxyethane, followed by treating with an alkali such as sodium hydroxide, etc. When the protecting group is an acid amide, the removal thereof is carried out by reacting in the presence of an acid such as hydrochloric acid, sulfuric acid, etc. in a solvent such as water, aqueous methanol, aqueous tetrahydrofuran, etc.

The compound (1) may include ones having an optical asymmetric center, and these compounds may be obtained in the form of a racemic mixture or in the form of an optically active compound when an optically active starting compound is used. If necessary, the obtained racemic mixture can be physically or chemically resolved into optical chiral compounds thereof by a conventional method, and preferably a racemic mixture is resolved into a diastereomer thereof by a reaction of using an optical active resolving agent. Other diastereomers are also resolved by a conventional method such as fractional crystallization.

The "prodrug" includes a compound, which can easily be hydrolyzed in the living body, and can reproduce the compound of the formula (1). The "prodrug" is, for example, when such a compound of the formula (1) has a carboxyl group, then ones wherein said carboxyl group is replaced by an alkoxycarbonyl group, an alkylthiocarbonyl group, or an alkylaminocarbonyl group, or when a compound of the formula (1) has an amino group, then ones wherein said amino group is substituted by an alkanoyl group to form an alkanoylamino group, or substituted by an alkoxycarbonyl group to form an alkoxycarbonylamino group, or converted into an acyloxymethylamino group or a hydroxylamine. When a compound of the formula (1) has a hydroxy group, the prodrug thereof is, for example, compounds wherein said hydroxy group is substituted by an acyl group as mentioned above and converted into an acyloxy group, or converted into a phosphate ester, or converted into an acyloxymethyloxy group. The alkyl moiety of groups being used for making a prodrug may be the above-mentioned alkyl groups, and said alkyl group may optionally be substituted, for example, by an alkoxy group having 1 to 6 carbon atoms, etc. The preferable example are, for example, in the compounds wherein a carboxyl group is converted into an alkoxycarbonyl group, a lower (e.g., having 1 to 6 carbon atoms) alkoxycarbonyl group such as methoxycarbonyl, ethoxycarbonyl, etc., or a lower (e.g., having 1 to 6 carbon atoms) alkoxycarbonyl group being substituted by an alkoxy group such as methoxymethoxycarbonyl, ethoxymethoxycarbonyl, 2-methoxyethoxycarbonyl, 2-methoxyethoxymethoxycarbonyl, or pivaloyloxymethoxycarbonyl.

The compound (1) or a prodrug thereof can be converted into a pharmaceutically acceptable salt thereof, if necessary. The pharmaceutically acceptable salt includes, for example, a salt with an inorganic acid such as hydrochloric acid, hydrobromic acid, sulfuric acid, phosphoric acid, etc.; or a salt with an organic carboxylic acid such as formic acid, acetic acid, fumaric acid, maleic acid, oxalic acid, citric acid, malic acid, tartaric acid, aspartic acid, glutamic acid, etc.; a salt with a sulfonic acid such as methanesulfonic acid, benzenesulfonic acid, p-toluenesulfonic acid, hydroxybenzensulfonic acid, dihydroxybenzenesulfonic acid, etc.; an alkali metal salt such as sodium salt, potassium

salt, etc.; an alkaline earth metal salt such as calcium salt, magnesium salt, etc.; ammonium salt; triethylamine salt; a pyridine salt; a picoline salt; an ethanolamine salt; a dicyclohexylamine salt; a salt with N,N'-dibenzylethylenediamine, etc.

The present compound (1) or a prodrug thereof, or a pharmaceutically acceptable salt of the same may be in the form of an anhydrous product thereof, or in the form of a hydrate or a solvate.

The present compounds can be administered either parenterally or orally when used as a medicament. The present compounds can be orally administered in the form of a conventional administration form such as powders, granules, tablets, capsules, syrups, suspensions, etc., or parenterally administered in the form of an injection such as solutions, emulsions, suspensions, etc., or can be administered rectally in the form of a suppository. The above suitable preparations can be formulated, for example, by mixing the present compound with a conventional carrier, excipient, binder, stabilizer, diluent. When the present compounds are used in the form of an injection, they may additionally contain a pharmaceutically acceptable buffering agent, solubilizer and isotonic agent. The dosage and the frequency of administration of the present compounds may vary according to the diseases to be cured, the conditions, ages, weights of the patients and the administration form, but the present compounds can usually be administered in a dose of 0.1 to 2000 mg per day, preferably in a dose of 1 to 200 mg per day in adult, once a day, or divided into several times (e.g., 2 - 4 times).

EXAMPLES

The present invention is illustrated in more detail by the following Examples and Experiment, but should not be construed to be limited thereto. In addition, the chemical names used in the following Examples and Experiment are not always ones based on the nomenclature by IUPAC.

Example 1

Preparation of 5,6-dihydro-1H-pyrrolo[3,2,1-ij]quinazoline-1,3(2H)-dione:

(1) 7-Indolinecarboxamide

To a mixture of 1-(tert-butoxycarbonyl)-7-indolinecarboxylic acid (3.67 g, 13.9 mmol), chloroform (30 mL) and a drop of dimethyl formamide was added dropwise with stirring oxalyl chloride (5.5 mL, 63.0 mmol) over a period of 5 minutes at room temperature. After the addition, the reaction mixture was warmed to 60°C, and stirred at 60°C for 2 hours. The reaction mixture was concentrated under reduced pressure, and thereto was added toluene (about 100 mL), and the mixture was further concentrated under reduced pressure. To the residue was added tetrahydrofuran (80 mL), and thereto was added with stirring a 29 % aqueous ammonia (55 mL) with stirring under ice-cooling. The mixture was warmed to room temperature and stirred overnight. Water was added to the reaction mixture, and the mixture was extracted with chloroform. The organic layer was washed with a saturated brine, dried over anhydrous magnesium sulfate, filtered, and concentrated. The resulting residue was purified by silica gel column chromatography ($SiO_2$, hexane : ethyl acetate = 1:2, and then ethyl acetate) to give 7-indolinecarboxamide (2.02 g, yield: 90 %).

$^1H$ NMR (DMSO-$d_6$) δ; 7.62 (br, 1H), 7.36 (d, 1H, J=8.1Hz), 7.08 (d, 1H, J=7.0Hz), 7.00 (br, 1H), 6.61 (s, 1H), 6.46-6.41 (m, 1H), 3.52 (t, 2H, J=8.5 Hz), 2.90 (t, 2H, J=8.5 Hz)

(2) 5,6-Dihydro-1H-pyrrolo[3,2,1-ij]quinazoline-1,3(2H)-dione

A mixture of 7-indolinecarboxamide (62.6 mg, 0.386 mmol), N,N-carbonyldiimidazole (300.1 mg, 1.85 mmol) and tetrahydrofuran (2 mL) was heated with stirring under reflux for 7.5 hours. The reaction mixture was cooled to room temperature, and thereto was added gradually a 5 % aqueous potassium hydrogen sulfate solution, and the mixture was extracted with chloroform. The organic layer was washed with a saturated brine, dried over anhydrous magnesium sulfate, filtered, and concentrated. The resulting residue was purified by silica gel column chromatography ($SiO_2$, ethyl acetate) to give 5,6-dihydro-1H-pyrrolo[3,2,1-ij]quinazoline-1,3(2H)-dione (50.8 mg, yield: 70 %).

M.p.: 314-315°C

The compound of Example 2 was obtained in a similar manner as in Example 1.

Example 2

6,7-Dihydro-1H,5H-pyrido (3,2,1-ij]quinazoline-1,3(2H)-dione:

M.p.: 282-284°C

Example 3

Preparation of 5-(hydroxymethyl)-6,7-dihydro-1H,5H-pyrido[3,2,1-ij]-quinazoline- 1,3(2H)-dione:

(1) 2-({[tert-Butyl(dimethyl)silyl]oxy}methyl)-1,2,3,4-tetrahydroquinoline

To a mixture of 1,2,3,4-tetrahydro-2-quinolinylmethanol (18.60 g, 0.114 mol), imidazole (8.54 g, 0.125mol) and dimethylformamide (10 mL) was added with stirring tent-butyldimethylsilyl chloride (18.03 g, 0.120 mol) under ice-cooling, and after the ice bath was removed, the mixture was stirred at room temperature overnight. To the reaction mixture was added cold 2 % aqueous potassium hydrogen sulfate solution, and the mixture was extracted with diethyl ether. The organic layer was washed successively with water and a saturated brine, and dried over anhydrous magnesium sulfate, filtered and concentrated to give 2-({[tert-butyl(dimethyl)silyl]oxy}methyl)-1,2,3,4-tetrahydroquinoline (32.36 g) as a crude product.

$^1$H NMR ($CDCl_3$) δ; 6.99-6.94 (m, 2H), 6.62-6.58 (m, 1H), 6.51 (d, 1H, J=7.9Hz), 4.27 (br s, 1H), 3.69 (dd, 1H, J=4.0, 9.4Hz), 3.51-3.35 (m, 2H), 2.9-2.7 (m, 2H), 1.9-1.8 (m, 1H), 1.64-1.51 (m, 1H), 0.93 (s, 9H), 0.09 (s, 3H), 0.08 (s, 3H)

(2) tert-Butyl 2-({[tert-butyl(dimethyl)silyl]oxy}methyl)-3,4-dihydro-1(2H)-quinoline carboxylate

A mixture of crude 2-({[tert-butyl(dimethyl)silyl]oxy}methyl)-1,2,3,4-tetrahydroquinoline (32.35 g), di-tert-butyl dicarbonate (28.8 mL, 0.125mol) and tetrahydrofuran (80 mL) was heated with stirring under reflux. Eight hours after the reflux began, di-tert-butyl dicarbonate (5.0 mL, 0.022 mol) was added to the mixture, and the mixture was further heated with stirring under reflux for 25 hours. The reaction mixture was concentrated, and the resulting residue was purified by silica gel column chromatography ($SiO_2$, hexane : ethyl acetate = 30:1, and then 20:1) to give crude tert-butyl 2-({[tert-butyl-(dimethyl)silyl]oxy}methyl)-3,4-dihydro- 1 (2H)-quinolinecarboxylate containing di-tert-butyl dicarbonate (45.43 g).

$^1$H NMR ($CDCL_3$) δ; 7.47 (d, 1H, J=7.9Hz), 7.15-6.96 (m, 3H), 4.53-4.44 (m, 1H), 3.65 (dd, 1H, J=5.1, 9.8Hz), 3.50 (dd, 1H, J=7.1, 9.8Hz), 2.65-2.60 (m, 2H), 2.25-2.14 (m, 1H), 1.84-1.72 (m, 1H), 1.51 (s, 9H), 0.81 (s, 9H), -0.01 (s, 3H), -0.03 (s, 3H)

(3) 1-(tert-Butoxycarbonyl)-2-({[tert-butyl(dimethyl)silyl]oxy}methyl)-1,2,3,4-tetrahydro-8-quinolinecarboxylic acid

To a mixture of crude tert-butyl 2-({[tert-butyl(dimethyl)silyl]oxy}-methyl)-3,4-dihydro-1(2H)-quinolinecarboxylate (42.78 g, tetramethylethylenediamine (21.4 mL, 0.142 mol) and diethyl ether (400 mL) was added dropwise with stirring a 0.96 mol/L solution of sec-butyllithium in cyclohexane/n-hexane solution (134 mL, 0.129 mol) at -78°C over a period of 40 minutes, and further stirred at -78°C for one hour. Dry ice (about 150 g) was added to the mixture with stirring at -78°C, and after the dry ice bath was removed, the mixture was stirred while the mixture was gradually warmed to room temperature over a period of one hour. The mixture was allowed to stand at room temperature overnight. The reaction mixture was poured into a 5 % aqueous potassium hydrogen sulfate solution/ice, and the mixture was extracted with ethyl acetate. The organic layer was washed with a saturated brine, dried over anhydrous magnesium sulfate, filtered, and concentrated. To the resulting residue were added diethyl ether and hexane, and the resulting solid was collected by fitration to give 1-(tert-butoxycarbonyl)-2-({(tert-butyl(dimethyl)silyl]oxy}methyl)-1,2,3,4-tetrahydro-8-quinooinecarboxylic acid (26.79 g) as a primary crystal. The filtrate was further concentrated and treated likewise to give 1-(tert-butoxycarbonyl)-2-({(tert-butyl(dimethyl) silyl]oxy}methyl)-1,2,3,4-tetrahydro-8-quinooinecarboxylic acid (3.00 g) as a secondary crystal (totally 66 % yield from 1,2,3,4-tetrahydro-2-quinolinylmethanol).

$^1$H NMR (DMSO-$d_6$) δ; 12.49 (br s, 1H), 7.56 (d, 1H, J=7.7Hz), 7.30 (dd, 1H, J=1.4, 7.4Hz), 7.12-7.07 (m, 1H), 4.4-4.25 (m, 1H), 3.85 (dd, 1H, J=3.6, 9.4Hz), 3.28-3.22 (m, 1H), 2.64-2.59 (m, 1H), 2.4-2.25 (m, 2H), 1.43-1.29 (m, 10H), 0.75 (s, 9H), -0.02 (s, 3H), -0.06 (s, 3H).

(4) tert-Butyl 8-(aminocarbonyl)-2-({[tert-butyl(dimethyl)silyl]oxy}-methyl)-3,4-dihydro- 1(2H)-quinolinecarboxylate

To a mixture of 1-(tert-butoxycarbonyl)-2-({tert-butyl(dimethyl)-silyl]oxy}methyl)-1,2,3,4-tetrahydro-8-quinooinecarboxylic acid (12.63 g, 30.0 mmol), WSC hydrochloride (8.61 g, 44.9 mmol), 1-hydroxybenzotriazole (6.07 g, 44.9 mmol), ammonium chloride (3.25 g, 60.8 mmol) and dimethylformamide (80 mL) was added with stirring diisopropylethylamine (21.0 mL, 0.121 mol) at room temperature, and the mixture was stirred at room temperature overnight. The reaction mixture was poured into a 5 % aqueous potassium hydrogen sulfate solution/ice, and the mixture was extracted with diethyl ether. The organic layer was washed successively with a saturated aqueous sodium hydrogen carbonate solution and a saturated brine, dried over anhydrous magnesium sulfate, filtered, and concentrated. The resulting

residue was purified by silica gel column chromatography ($SiO_2$, hexane : ethyl acetate = 3:1, and then 1:1) to give tert-butyl 8-(aminocarbonyl)-2-({[tert-butyl-(dimethyl)silyl]oxy}methyl)-3,4-dihydro-1 (2H)-quinoline-carboxylate (10.26 g, yield: 81 %).

$^1$H NMR (DMSO-$d_6$) δ; 7.45 (br, 1H), 7.41 (dd, 1H, J=1.7, 7.7Hz), 7.23-7.20 (m, 2H), 7.12-7.07 (m, 1H), 4.43 (m, 1H), 3.85-3.78 (m, 1H), 3.51 (dd, 1H, J=4.5, 10.9Hz), 2.64-2.57 (m, 1H), 2.4-2.19 (m, 2H), 1.45-1.4 (m, 1H), 1.40 (s, 9H), 0.67 (s, 9H), 0.02 (s, 3H), -0.08 (s, 3H)

(5) 5-({[tert-Butyl(dimethyl) silyl]oxy}methyl)-6,7-dihydro-1H,5H-pyrido[3,2,1 -ij]quinazoline-1,3(2H)-dione

To a solution of tert-butyl-8-(aminocarbonyl)-2-({[tert-butyl-(dimethyl)silyl]oxy}methyl)-3,4-dihydro-1(2H)-quinolinecarboxylate (5.04 g, 12.0 mmol) in tetrahydrofuran (30 mL) was added with stirring a 60 % sodium hydride (1.20 g, 30.0 mmol) at room temperature, and then, the mixture was warmed to 50°C, and stirred at 50°C for 1.5 hour. The reaction mixture was poured into an aqueous ammonium chloride solution/ice, and the mixture was extracted with chloroform. The organic layer was dried over anhydrous magnesium sulfate, filtered, and concentrated. The resulting residue was purified by silica gel column chromatography ($SiO_2$, hexane : ethyl acetate = 1:1) to give 5-({[tert-butyl (dimethyl)silyl]oxy}methyl)-6,7-dihydro- 1H,5H-pyrido[3,2,1-ij]quinazoline-1,3(2H)-dione (3.82 g, yield: 92 %).

$^1$H NMR (DMSO-$d_6$) δ; 11.54 (s, 1H), 7.80 (d, 1H, J=7.OHz), 7.50 (d, 1H, J=7.3Hz), 7.16-7.11 (m, 1H), 4.66-4.61 (m, 1H), 3.72 (d, 2H, J=5.9Hz), 3.08-2.96 (m, 1H), 2.85-2.77 (m, 1H), 2.30-2.23 (m, 1H), 1.90-1.78 (m, 1H), 0.72 (s, 9H), -0.02 (s, 3H), -0.12 (s, 3H)

(6) 5-(Hydroxymethyl)-6,7-dihydro-1H,5H-pyrido[3,2,1-ij]-quinazoline-1,3(2H)-dione

A mixture of 5-({[tert-butyl(dimethyl)silyl]oxy}methyl)-6,7-dihydro-1H,5H-pyrido[3,2,1-ij]quinazoline-1,3(2H)-dione (3.65 g, 10.5 mmol),a 1 mol/L solution of tetrabutylammonium fluoride in tetrahydrofuran (11.6 mL, 11.6 mmol) and tetrahydrofuran (20 mL) was stirred at room temperature for 4 hours. The reaction mixture was poured into a 5 % potassium hydrogen sulfate, and the mixture was extracted with chloroform. The organic layer was washed with a saturated brine, dried over anhydrous magnesium sulfate, filtered, and concentrated. To a portion (3.18 g) of the resulting crude product (3.20 g) were added ethyl acetate and hexane, and the mixture was stirred, and the solid was collected by filtration. This solid was stirred in a mixture of methanol and water, and collected by filtration, and washed with water to give 5-(hydroxymethyl)-6,7-dihydro-1H,5H-pyrido[3,2,1-ij]quinazoline- 1,3(2H)-dione (1.91 g). Further, water was added to the filtration to give 5-(hydroxymethyl)-6,7-dihydro-1H,5H-pyrido[3,2,1-ij]-quinazoline-1,3(2H)-dione (0.20 g, total yield: 87 %).

M.p.: 205-207°C

The compound of Example 4 was obtained in a similar manner as in Example 3.

Example 4

5-(Hydroxymethyl)-5,6-dihydro- 1H-pyrrolo[3,2,1-ij]quinazoline-1,3(2H)-dione:

M.p.: 238-240°C

The compound of Example 5 was obtained in a similar manner as in Example 3.

Example 5

6-(Hydroxymethyl]-6,7-dihydro-1H,5H-pyrido[3,2,1-ij]quinazoline-1,3(2H)-dione:

M.p.: 252-254°C

The compound of Example 6 was obtained in a similar manner as in Example 3.

Example 6

7-(Hydroxymethyl)-6,7-dihydro- 1H,5H-pyrido [3,2,1-ij]quinazoline-1,3(2H)-dione:

M.p.: 226°C (decomposed)

Example 7

Preparation of 5-(bromomethyl)-6,7-dihydro-1H,5H-pyrido[3,2,1-ij]-quinazoline-1,3(2H)-dione:

To a mixture of 5-(hydroxymethyl)-6,7-dihydro-1H,5H-pyrido-(3,2,1-ij]quinazoline-1,3(2H)-dione (183.4 mg, 0.790 mmol), carbon tetrabromide (340.7 mg, 1.03 mmol) and acetonitrile (6 mL) was added with stirring triphenylphosphine (269.0 mg, 1.03 mmol) under ice-cooling. After the ice bath was removed, the mixture was stirred at room temperature for 5 hours. The reaction solution was cooled with ice, and the solid was collected by filtration, washed with ice-cooled acetonitrile to give 5-(bromomethyl)-6,7-dihydro-1H,5H-pyrido[3,2,1-ij]-quinazoline-1,3(2H)-dione (178.9 mg). The filtrate was concentrated to a half volume thereof and cooled with ice to give 5-(bromomethyl)-6,7-dihydro-1H,5H-pyrido(3,2,1-ij]quinazoline-1,3(2H)-dione (33.2 mg, total yield: 91 %).

$^1$H NMR (DMSO-$d_6$) δ; 11.63 (s, 1H), 7.83 (d, 1H, J=7.9Hz), 7.55 (d, 1H, J=7.7Hz), 7.20-7.15 (m, 1H), 4.81 (m, 1H), 3.60-3.53 (m, 2H), 3.03-2.78 (m, 2H), 2.46-2.40 (m, 1H), 1.94-1.81 (m, 1H)

The compound of Example 8 was obtained in a similar manner as in Example 7.

Example 8

5-(Bromomethyl)-5,6-dihydro-1H-pyrrolo[3,2,1-ij]quinazoline-1,3(2H)-dione

$^1$H NMR (DMSO-$d_6$) δ; 11.32 (s, 1H), 7.60 (d, 1H, J=8.1Hz), 7.56 (d, 1H, J=7.3Hz), 7.17-7.12 (m, 1H), 5.07-5.00 (m, 1H), 4.24-4.19 (m, 1H), 3.95-3.90 (m, 1H), 3.63-3.54 (m, 1H), 3.19-3.12 (m, 1H)

The compound of Example 9 was obtained in a similar manner as in Example 7.

Example 9

6-(Bromomethyl)-6,7-dihydro- 1H,5H-pyrido[3,2,1-ij]quinazoline-1,3(2H)-dione

M.p.: 267-270°C (decomposed)

The compound of Example 10 was obtained in a similar manner as in Example 7.

Example 10

7-(Bromomethyl)-6,7-dihydro-1H,5H-pyrido[3,2,1-ij]quinazoline-1,3(2H)-dione

M.p.: 225°C (decomposed)

Example 11

Preparation of 5-[(dimethylamino)methyl]-6,7-dihydro-1H,5H-pyrido-[3,2,1-ij]quinazoline-1,3(2H)-dione:

A mixture of 5-(bromomethyl)-6,7-dihydro-1H,5H-pyrido[3,2,1-ij]quinazoline-1,3(2H)-dione (164.4 mg, 0.557 mmol), dimethylamine (40 % aqueous solution, 1 mL) and dimethylformamide (2 mL) was stirred at room temperature overnight. To the reaction solution was added a 5 % aqueous potassium carbonate solution, and the mixture was extracted with chloroform. The organic layer was washed with a saturated brine, dried over anhydrous sodium sulfate, filtered, and concentrated. The resulting residue was purified by thin layer chromatography ($SiO_2$, chloroform : methanol: triethylamine = 40:2:1) to give 5-[(dimethylamino)methyl]-6,7-dihydro-1H,5H-pyrido[3,2,1-ij]-quinazoline-1,3(2H)-dione (31.8 mg, yield: 22 %).

$^1$H NMR (DMSO-$d_6$) δ; 11.48 (s, 1H), 7.81 (d, 1H, J=6.8Hz), 7.53 (d, 1H, J=7.3Hz), 7.18-7.13 (m, 1H), 4.78 (m, 1H), 2.98-2.74 (m, 2H), 2.42-2.14 (m, 3H), 2.19 (s, 6H), 1.78-1.66 (m, 1H)

M.p. of the hydrochloride: 295-297°C (decomposed)

The compound of Example 12 was obtained in a similar manner as in Example 11.

Example 12

6-[(Dimethylamino)methyl]-6,7-dihydro- 1H,5H-pyrido[3,2,1-ij]-quinazoline-1,3(2H)-dione

$^1$H NMR (DMSO-$d_6$) δ; 11.48 (s, 1H), 7.79 (d, 1H, J=7.9Hz), 7.51 (d, 1H, J=7.2Hz), 7.16-7.11 (m, 1H), 4.29-4.25 (m, 1H), 3.32-3.24 (m, 3H), 2.96-2.91 (m, 1H), 2.61-2.52 (m, 1H), 2.21 (m, 1H), 2.16 (s, 6H)

The compound of Example 13 was obtained in a similar manner as in Example 11.

Example 13

5-[(Dimethylamino)methyl]-5,6-dihydro- 1H-pyrrolo[3,2,1-ij]quinazoline-1,3(2H)-dione

$^1$H NMR (DMSO-$d_6$) δ; 11.19 (s, 1H), 7.59-7.53 (m, 2H), 7.16-7.11 (m, 1H), 4.77-4.69 (m, 1H), 3.47-3.38 (m, 1H), 3.31-3.24 (m, 1H), 2.78-2.73 (m, 1H), 2.5-2.43 (m, 1H), 2.18 (s, 6H)

M.p. of the hydrochloride: >300°C

The compound of Example 14 was obtained in a similar manner as in Example 11.

Example 14

7-[(Dimethylamino)methyl]-6,7-dihydro- 1H,5H-pyrido[3,2,1-ij]-quinazoline-1,3(2H)-dione

$^1$H NMR (DMSO-$d_6$) δ; 11.48 (s, 1H), 7.84-7.81 (m, 1H), 7.61-7.58 (m, 1H), 7.18-7.13 (m, 1H), 4.17-4.09 (m, 1H), 3.60-3.51 (m, 1H), 3.12-3.04 (m, 1H), 2.5-2.43 (m, 1H), 2.27-2.2 (m, 1H), 2.20 (s, 6H), 2.13-2.04 (m, 1H), 1.90-1.79 (m, 1H)

M.p. of the hydrochloride: >300°C

The following compound was also obtained as a by-product. 7-Methylene-6,7-dihydro- 1H,5H-pyrido[3,2,1-ij] quinazoline-1,3(2H)-dione

M.p.: 242-245°C

The compound of Example 15 was obtained in a similar manner as in Example 11.

Example 15

6-(1-Pyrrolidinylmethyl)-6,7-dihydro-1H,5H-pyrido[3,2,1-ij]quinazoline-1,3(2H)-dione

M.p. of the hydrochloride: >300°C

Example 16

Preparation of 1,3-dioxo-2,3,6,7-tetrahydro-1H,5H-pyrido[3,2,1-ij]-quinazoline-5-carbaldehyde:

To a mixture of 5-(hydroxymethyl)-6,7-dihydro-1H,5H-pyrido-[3,2,1-ij]quinazoline-1,3(2H)-dione (234.2 mg, 1.01 mmol), triethylamine (1.0 mL, 7.17 mmol) and dimethylsulfoxide (2.0 mL) was added with stirring sulfur trioxide pyridine complex (805.0 mg, 5.06 mmol) at room temperature, and the mixture was stirred at room temperature for one hour. The reaction solution was cooled with ice and thereto was added a 5 % aqueous potassium hydrogen sulfate solution, and extracted with chloroform. The organic layer was washed with a saturated brine, dried over anhydrous sodium sulfate, filtered, and concentrated. Water was added to the resulting residue, and the solid was collected by filtration, and washed with water to give 1,3-dioxo 2,3,6,7-tetrahydro-1H,5H-pyrido[3,2,1-ij]quinazoline-5-carbaldehyde (49.4 mg, yield: 21 %).

$^1$H NMR (DMSO-$d_6$) δ; 11.69 (s, 1H), 9.59 (s, 1H), 7.85 (d, 1H, J=7.7Hz), 7.50 (d, 1H, J=7.3Hz), 7.20-7.15 (m, 1H), 5.25-5.21 (m, 1H), 2.90-2.84 (m, 1H), 2.62-2.45 (m, 2H), 2.06-1.96 (m, 1H)

Example 17

Preparation of 6-(aminomethyl)-6,7-dihydro- 1H,5H-pyrido[3,2,1-ij]-quinazoline-1,3(2H)-dione hydrochloride:

(1) 6-(Azidomethyl)-6,7-dihydro-1H,5H-pyrido[3,2,1-ij]quinazoline-1,3(2H)-dione

A mixture of 6-(bromomethyl)-6,7-dihydro-IH,SH-pyrido[3,2,1-ij]quinazoline-1,3(2H)-dione (302.2 mg, 1.02 mmol), sodium azide (600.3 mg, 9.23 mmol) and dimethylformamide (3 mL) was stirred at 60°C for 2 hours. Water was added to the reaction solution, and the mixture was stirred under ice-cooling. The precipitated solid was collected by filtration, and washed with cold water to give 6-(azido-methyl)-6,7-dihydro- 1H,5H-pyrido[3,2,1-ij]quinazoline-1,3 (2H)-dione (237.4 mg, yield: 90 %).

$^1$H NMR (DMSO-$d_6$) δ; 11.51 (s, 1H), 7.80 (d, 1H, J=7.5Hz), 7.53 (d, 1H, J=6.8Hz), 7.18-7.13 (m, 1H), 4.23-4.18 (m, 1H), 3.56-3.40 (m, 3H), 3.02-2.95 (m, 1H), 2.73-2.64 (m, 1H), 2.24 (m, 1H)

(2) 6-(Aminomethyl)-6,7-dihydro- 1H,5H-pyrido[3,2,1-ij]quinazoline-1,3(2H)-dione hydrochloride

To a mixture of 6-(azidomethyl)-6,7-dihydro-1H,5H-pyrido[3,2,1-ij]quinazoline-1,3(2H)-dione (200.4 mg, 0.779 mmol), tetrahydrofuran (4 mL) and water (1.5 mL) was added with stirring triphenylphosphine (214.8 mg, 0.819 mmol) at room temperature, and the mixture was stirred at 60°C for 1.5 hour, and heated under reflux for 40 minutes. To the mixture was added triphenylphosphine (46.6 mg, 0.174 mmol), and the mixture was further heated under reflux for one hour. The reaction mixture was concentrated under reduced pressure, and 1N aqueous hydrochloric acid solution and ethyl acetate were added to the resulting residue for separation. The hydrochloric acid layer was concentrated under reduced pressure to give 6-(aminomethyl)-6,7-dihydro-1H,5H-pyrido[3,2,1-ij]quinazoline-1,3(2H)-dione hydrochloride (203.5 mg, yield: 98 %).

M.p.: >300°C

The compound of Example 18 was obtained in a similar manner as in Example 17.

Example 18

5-(Aminomethyl)-6,7-dihydro- 1H,5H-pyrido(3,2,1-ij]quinazoline-1,3(2H)-dione hydrochloride

M.p.: >300°C

The compound of Example 19 was obtained in a similar manner as in Example 17.

Example 19

5-(Aminomethyl)-5,6-dihydro-1H-pyrrolo[3,2,1-ij]quinazoline-1,3(2H)-dione hydrochloride

M.p.: >300°C

The compound of Example 20 was obtained in a similar manner as in Example 17.

Example 20

7-(Aminomethyl)-6,7-dihydro- 1H,5H-pyrido(3,2,1-ij]quinazoline-1,3(2H)-dione hydrochloride

M.p.: 190°C (decomposed)

Example 21

Preparation of N-[(1,3-dioxo-2,3,6,7-tetrahydro-1H,5H-pyrido[3,2,1-ij]-quinazolin-6-yl)methyl]benzamide:

A mixture of 6-(aminomethyl)-6,7-dihydro-1H,5H-pyrido[3,2,1-ij]quinazoline-1,3(2H)-dione hydrochloride (282.6 mg, 1.06 mmol), WSC hydrochloride (262.1 mg, 1.37 mmol), 1-hydroxybenzotriazole (158.0 mg, 1.17 mmol), benzoic acid (129.3 mg, 1.06 mmol), triethylamine (0.37 mL, 2.65 mmol) and dimethylformamide (5 mL) was stirred at room temperature overnight. To the reaction mixture was added a 5 % aqueous potassium hydrogen sulfate solution, and the mixture was extracted with chloroform. The organic layer was washed successively with a saturated aqueous sodium hydrogen carbonate solution and a saturated brine, dried over anhydrous magnesium sulfate, filtered, and concentrated. The resulting residue was stirred in a small amount of chloroform, and the solid was collected by filtration, and washed with a small amount of chloroform to give N-[(1,3-dioxo-2,3,6,7-tetrahydro-1H,5H-pyrido[3,2,1-ij]quinazoline-6-yl)methyl]benzamide (283.7 mg, yield: 80 %).

M.p.: 277-280°C

The compound of Example 22 was obtained in a similar manner as in Example 21.

Example 22

N-[(1,3-Dioxo-2,3,6,7-tetrahydro- 1H,5H-pyrido[3,2,1-ij]quinazolin-6-yl) methyl]acetamide

$^1H$ NMR (DMSO-$d_6$)δ; 11.48 (s, 1H), 8.06 (t, 1H, J=5.5Hz), 7.80 (d, 1H, J=6.6Hz), 7.51 (d, 1H, J=7.5Hz), 7.17-7.12 (m, 1H), 4.22-4.17 (m, 1H), 3.36-3.28 (m, 1H), 3.20-3.03 (m, 2H), 2.95-2.88 (m, 1H), 2.65-2.57 (m, 1H), 2.13-2.02 (m, 1H), 1.83 (s, 3H)

The compound of Example 23 was obtained in a similar manner as in Example 21.

Example 23

N-[(1,3-Dioxo-2,3,6,7-tetrahydro- 1H,5H-pyrido[3,2,1-ij]quinazolin-6-yl)-methyl]isonicotinamide

M.p.: 282-285°C

The compound of Example 24 was obtained in a similar manner as in Example 21.

Example 24

N-[(1,3-Dioxo-2,3,6,7-tetrahydro- 1H,5H-pyrido[3,2,1-ij]quinazolin-6-yl)-methyl]-2-pyridinecarboxamide

M.p.: 247-250°C

The compound of Example 25 was obtained in a similar manner as in Example 21.

Example 25

N-[(1,3-Dioxo-2,3,6,7-tetrahydro-1H,5H-pyrido[3,2,1-ij]quinazolin-6-yl)-methyl]nicotinamide

$^1H$ NMR (DMSO-$d_6$) δ; 11.46 (s, 1H),9.01 (d,1H, J=1.7Hz), 8.87 (t, 1H, J=5.6Hz), 8.70 (dd, 1H, J=1.7, 4.8Hz), 8.20-8.16 (m, 1H), 7.81 (dd, 1H, J=1.4, 7.8Hz), 7.54-7.49 (m, 2H), 7.18-7.12 (m, 1H), 4.24-4.19 (m, 1H), 3.51-3.3 (m, 3H), 3.04-2.97 (m, 1H), 2.75-2.67 (m, 1H), 2.35-2.25 (m, 1H)

M.p.: >300°C

The compound of Example 26 was obtained in a similar manner as in Example 21.

Example 26

N-[(1,3-Dioxo-2,3,6,7-tetrahydro- 1H,5H-pyrido[3,2,1-ij]quinazolin-6-yl) methyl]-2-phenylacetamide

M.p.: 275°C (decomposed)

The compound of Example 27 was obtained in a similar manner as in Example 21.

Example 27

2-(Dimethylamino)-N-[(1,3-dioxo-2,3,6,7-tetrahydro-1 H,5H-pyrido[3,2,1-ij]quinazolin-6-yl)methyl]benzamide

M.p.: 220-236°C

The compound of Example 28 was obtained in a similar manner as in Example 21.

Example 28

3-(Dimethylamino)-N-[(1,3-dioxo-2,3,6,7-tetrahydro-1H,5H-pyrido[3,2,1-ij]quinazolin-6-yl)methyl]benzamide

M.p.: 158-170°C

The compound of Example 29 was obtained in a similar manner as in Example 21.

Example 29

4-(Dimethylamino)-N-[[1,3-dioxo-2,3,6,7-tetrahydro-1H,5H-pyrido[3,2,1-ij]quinazolin-6-yl)methyl]benzamide

M.p.: 268-278°C (decomposed)

The compound of Example 30 was obtained in a similar manner as in Example 21.

Example 30

N-[(1,3-Dioxo-2,3,6,7-tetrahydro-1H,5H-pyrido[3,2,1-ij)quinazolin-6-yl)-methyl]cyclohexanecarboxamide

M.p.: 280-281°C (decomposed)

The compound of Example 31 was obtained in a similar manner as in Example 21.

Example 31

N-[(1,3-Dioxo-2,3,6,7-tetrahydro- H,5H-pyrido[3,2,1-ij]quinazolin-6-yl)-methyl]-2-pyrazinecarboxamide

M.p.: 255-257°C (decomposed)
The compound of Example 32 was obtained in a similar manner as in Example 21.

Example 32

N-[(1,3-Dioxo-2,3,6,7-tetrahydro- 1H,5H-pyrido[3,2,1-ij]quinazolin-6-yl)-methyl]-4-pyridazinecarboxamide

M.p.: 279-281°C (decomposed)
The compound of Example 33 was obtained in a similar manner as in Example 21.

Example 33

4-[(Dimethylamino)methyl]-N-[(1,3-dioxo-2,3,6,7-tetrahydro-1H,5H-pyrido[3,2,1 -ij]quinazolin-6-yl)methyl]benzamide

M.p.: 240-243°C (decomposed)
The compound of Example 34 was obtained in a similar manner as in Example 21.

Example 34

N-[(1,3-Dioxo-2,3,6,7-tetrahydro-1H,5H-pyrido[3,2,1-ij]quinazolin-6-yl)-methyl]-l 1-methyl-4-piperidinecarboxamide

M.p.: 246-250°C (decomposed)
The compound of Example 35 was obtained in a similar manner as in Example 21.

Example 35

1-Benzyl-N-[(1,3-dioxo-2,3,6,7-tetrahydro-1H,5H-pyrido(3,2,1-ij]-quinazolin-6-yl)methyl]-4-piperidinecarboxamide

M.p.: 240-242°C (decomposed)

Example 36

Preparation of $N^1$-[(1,3-dioxo-2,3,5,6-tetrahydro-1H-pyrrolo[3,2,1-ij]-quinazolin-5-yl)methyl]glycinamide hydrochloride

5-(Aminomethyl)-5,6-dihydro- 1H-pyrrolo[3,2,1-ij]quinazoline-1,3(2H)-dione hydrochloride and N-Boc-glycine were reacted in a similar manner as in Example 21, and the resulting product was deprotected to give the compound of Example 36.
M.p.: 293-300°C (decomposed)
The compound of Example 37 was obtained in a similar manner as in Example 36.

Example 37

$N^1$-[(1,3-Dioxo-2,3,5,6-tetrahydro- 1H-pyrrolo[3,2,1-ij]quinazolin-5-yl)-methyl]-β-alaninamide hydrochloride

M.p.: 290-296°C (decomposed)
The compound of Example 38 was obtained in a similar manner as in Example 36.

Example 38

4-(Aminomethyl)-N-[(1,3-dioxo-2,3,6,7-tetrahydro-1H,5H-pyrido[3,2,1-ij]quinazolin-6-yl)methyl]benzamide hydrochloride

M.p.: >300°C
The compound of Example 39 was obtained in a similar manner as in Example 36.

Example 39

N-[(1,3-Dioxo-2,3,6,7-tetrahydro- 1H,5H-pyrido[3,2,1-ij]quinazolin-6-yl)-methyl] -4-piperidinecarboxamide hydrochloride

$^1$H NMR (DMSO-$d_6$)δ; 11.49 (s, 1H), 8.87 (br, 1H), 8.49 (br, 1H), 8.19 (t, 1H, J=5.3Hz), 7.80 (d, 1H, J=7:9Hz), 7.50 (d, 1H, J=7.5Hz), 7.17-7.12 (m, 1H), 4.18-4.12 (m, 1H), 3.4-2.4 (m, 10H), 2.12 (m, 1H), 1.87-1.66 (m, 4H)

Example 40

Preparation of 1,3-dioxo-2,3,6,7-tetrahydro-1H,5H-pyrido[3,2,1-ij]-quinazolin-5-carboxylic acid:

To a solution of 5-(hydroxymethyl}-6,7-dihydro-1H,5H-pyrido-[3,2,1-ij]quinazoline-1,3(2H)-dione (113.4 mg, 0.488 mmol) in dimethylformamide (1.3 mL) was added with stirring pyridinium dichromate (643.5 mg, 1.71 mmol) at room temperature, and the mixture was stirred at room temperature overnight. To the reaction solution was added toluene, and the mixture was concentrated under reduced pressure. The resulting residue was purified by thin layer chromatography ($SiO_2$, chloroform : methanol : acetic acid = 50:5:1) to give 1,3-dioxo-2,3,6,7-tetrahydro- 1H,5H-pyrido {3,2,1-ij]quinazoline-5-carboxylic acid (45.7 mg, yield: 38 %).

M.p.: >300°C

Example 41

Preparation of 6-{[benzyl(methyl)amino]methyl}-6,7-dihydro-1H,5H-pyrido[3,2,1-ij]quinazoline-1,3(2H)-dione:

(1) 6-(Hydroxymethyl)-2-(4-methoxybenzyl)-6,7-dihydro- 1H,5H-pyrido(3,2,1-ij]quinazoline-1,3(2H)-dione

To a mixture of 6-(hydroxymethyl)-6,7-dihydro-1H,5H-pyrido-[3,2,1-ij]quinazoline-1,3(2H)-dione (1.00 g, 4.31 mmol), potassium carbonate (778.6 mg, 5.63 mmol) and dimethylformamide (10 mL) was added with stirring 4-methoxybenzyl chloride (0.630 mL, 4.65 mmol) at room temperature, and the mixture was stirred at 50°C. Three and half hours later, 4-methoxybenzyl chloride (0.070 mL, 0.52 mmol) was added to the mixture, and the mixture was further stirred at 50°C for one hour. The reaction solution was cooled with ice, and thereto was added water, and the mixture was extracted with chloroform. The organic layer was washed with a saturated brine, dried over anhydrous magnesium sulfate, filtered, and concentrated. The resulting residue was purified by silica gel column chromatography ($SiO_2$, hexane : ethyl acetate =1:1, and then ethyl acetate) to give 6-(hydroxymethyl)-2-(4-methoxybenzyl)-6,7-dihydro- 1H, 5H-pyrido[3,2,1-ij)quinazoline-1,3(2H)-dione (1.36 g, yield: 90 %).

$^1$H NMR ($CDCl_3$) δ; 7.88-7.85 (m, 1H), 7.55-7.51 (m, 1H), 7.31-7.26 (m, 2H), 7.17 (t, 1H, J=7.7Hz), 6.87-6.82 (m, 2H), 5.10-5.00 (m, 2H), 4.83 (t, 1H, J=5.2Hz), 4.37-4.32 (m, 1H), 3.70 (s, 3H), 3.54-3.36 (m, 3H), 2.94-2.87 (m, 1H), 2.67-2.86 (m, 1H), 2.15-2.01 (m, 1H)

(2) 6-(Bromomethyl)-2-(4-methoxybenzyl)-6,7-dihydro- 1H,5H-pyrido[3,2,1-ij]quinazoline-1,3(2H)-dione

To a mixture of 6-(hydroxymethyl)-2-(4-methoxybenzyl)-6,7-dihydro-lH,5H-pyrido[3,2,1-ij]quinazoline-1,3 (2H)-dione (860.1 mg, 2.44 mmol), carbon tetrabromide (1.29 g, 3.89 mmol) and acetonitrile (10 mL) was added with stirring triphenylphosphine (1.02 g, 3.89 mmol) under ice-cooling, and after the ice bath was removed, the mixture was stirred at room temperature overnight. The reaction solution was concentrated under reduced pressure, and the resulting residue was purified by silica gel column chromatography ($SiO_2$, hexane : ethyl acetate = 2:1, and then, 1:1) to give 6-(bromomethyl)-2-(4-methoxy-benzyl)-6,7-dihydro-11H,5H-pyrido[3,2,1-ij]quinazoline-1,3(2H)-dione (1.02 g, quantitatively).

$^1$H NMR ($CDCl_3$) δ; 8.07 (d, 1H, J=7.9Hz), 7.53-7.49 (m, 2H), 7.44-7.41 (m, 1H), 7.18-7.13 (m, 1H), 6.86-6.81 (m, 2H), 5.26-5.15 (m, 2H), 4.56-4.50 (m, 1H), 3.77 (s, 3H), 3.64-3.56 (m, 1H), 3.47 (d, 2H, J=6.2Hz), 3.11-3.03 (m, 1H), 2.89-2.80 (m, 1H), 2.49-2.36 (m, 1H).

(3) 6-{[Benzyl(methyl)amino]methyl}-2-(4-methoxybenzyl)-6,7-dihydro-1H,5H-pyrido[3,2,1-ij]quinazoline-1,3(2H)-dione

A mixture of 6-(bromomethyl)-2-(4-methoxybenzyl)-6,7-dihydro-1H,5H-pyrido[3,2,1-ij]quinazoline-1,3(2H)-dione (101.3 mg, 0.244 mmol), N-benzylmethylamine (0.15 mL, 1.16 mmol) and dimethylformamide (1 mL) was stirred at room temperature overnight. Toluene was added to the reaction solution, and the mixture was concentrated under

reduced pressure (thrice). To the resulting residue were added chloroform and anhydrous potassium carbonate, and the mixture was stirred for 20 minutes. The mixture was filtered and concentrated. This residue was purified by silica gel column chromatography ($SiO_2$, hexane : ethyl acetate = 2:1, and then, 1:1) to give 6-{[benzyl(methyl)amino]methyl}-2-(4-methoxybenzyl)-6,7-dihydro- 1H,5H-pyrido[3,2,1-ij]quinazoline-1,3(2H)-dione (85.5 mg, yield: 77 %).

$^1$H NMR ($CDCl_3$) δ; 8.03-8.00 (m, 1H), 7.54-7.49 (m, 2H), 7.39-7.36 (m, 1H), 7.31-7.20 (m, 5H), 7.13-7.08 (m, 1H), 6.85-6.80 (m, 2H), 5.26-5.15 (m, 2H), 4.48-4.42 (m, 1H), 3.76 (s, 3H), 3.49 (s, 2H), 3.47-3.40 (m, 1H), 3.04-2.97 (m, 1H), 2.60-2.51 (m, 1H), 2.38-2.25 (m, 3H), 2.24 (s, 3H)

(4) 6-{[Benzyl(methyl)amino]methyl}-6,7-dihydro-1H,5H-pyrido-[3,2,1-ij]quinazoline-1,3(2H)-dione

To a mixture of 6-{[benzyl(methyl)amino]methyl}-2-(4-methoxybenzyl)-6,7-dihydro-1H,5H-pyrido[3,2,1-ij] quinazoline-1,3(2H)-dione (85.4 mg, 0.187 mmol), acetonitrile (2.0 mL) and water (1.0 mL) was added with stirring ceric ammonium nitrate (392.8 mg, 0.717 mmol) at room temperature, and the mixture was stirred at room temperature for 5.5 hours. To the reaction solution was a saturated aqueous sodium hydrogen carbonate solution, and the mixture was extracted with chloroform. The organic layer was dried over anhydrous sodium sulfate, filtered, and concentrated. The residue was purified by thin layer chromatography ($SiO_2$, hexane : ethyl acetate = 1:1) to give 6-{[benzyl(methyl) ammo]methyl}-6,7-dihydro-1H,5H-pyrido[3,2,1-ij]-quinazoline-1,3(2H)-dione (28.1 mg, yield: 45 %).

$^1$H NMR (DMSO-$d_6$) δ; 11.47 (s, 1H), 7.75 (d, 1H, J=7.9Hz), 7.50-7.47 (m, 1H), 7.28-7.08 (m, 6H), 4.16-4.11 (m, 1H), 3.56-2.19 (m, 8H), 2.17(s, 3H)

The compound of Example 42 was obtained in a similar manner as in Example 41.

Example 42

6-{[Methyl(2-phenylethyl)amino]methyl}-6,7-dihydro- 1H,5H-pyrido-[3,2,1-ij]quinazoline-1,3(2H)-dione

$^1$H NMR (DMSO-$d_6$) δ; 11.47 (s, 1H), 7.79 (d, 1H, J=7.7Hz), 7.48-7.45 (m, 1H), 7.27-7.11 (m, 6H), 4.25-4.20 (m, 1H), 3.35-2.15 (m, 10H), 2.25 (s,3H)

Example 43

Preparation of 6-[(benzyloxy)methyl]-6,7-dihydro-1H,5H-pyrido[3,2,1-ij]-quinazoline-1,3(2H)-dione:

(1) 6-[(Benzyloxy) methyl]-2-(4-methoxybenzyl)-6,7-dihydro- 1H,5H-pyrido[3,2,1-ij]quinazoline-1,3(2H)-dione

To a mixture of 6-(hydroxymethyl)-2-(4-methoxybenzyl)-6,7-dihydro-1H,5H-pyrido[3,2,1-ij]quinazoline-1,3 (2H)-dione (111.6 mg, 0.317 mmol) and tetrahydrofuran (3 mL) was added with stirring a 60 % sodium hydride (14.0 mg, 0.35 mmol) at room temperature, and the mixture was stirred at 60°C for one hour. The reaction solution was cooled to room temperature, and thereto was added benzyl bromide (0.050 mL, 0.42 mmol) while it was stirred at room temperature. The mixture was stirred at room temperature for 30 minutes, stirred at 50°C for 30 minutes, and stirred at 60°C for 1.5 hour. To the mixture were added benzyl bromide (0.030 mL, 0.25 mmol) and a 60 % sodium hydride (7.9 mg, 0.20 mmol), and the mixture was further stirred at 60°C for one hour. The reaction solution was poured into a 5 % aqueous potassium hydrogen sulfate solution, and the mixture was extracted with ethyl acetate. The organic layer was washed with a saturated brine, dried over anhydrous magnesium sulfate, filtered, and concentrated. The resulting residue was purified by silica gel column chromatography ($SiO_2$, hexane : ethyl acetate = 4:1, and then 2:1) to give 6-[(benzyloxy)methyl]-2-(4-methoxybenzyl)-6,7-dihydro-1H,5H-pyrido[3,2,1-ij]quinazoline-1,3(2H)-dione (124.0 mg, yield: 88 %).

$^1$H NMR ($CDCl_3$) δ; 8.04 (d, 1H, J=7.2Hz), 7.53-7.48 (m, 2H), 7.40-7.29 (m, 6H), 7.14-7.09 (m, 1H), 6.85-6.80 (m, 2H), 5.25-5.16 (m, 2H), 4.53 (s, 2H), 4.52-4.46 (m, 1H), 3.76 (s, 3H), 3.59-3.50 (m, 3H), 3.00-2.94 (m, 1H), 2.81-2.72 (m, 1H), 2.44-2.28 (m, 1H)

(2) 6-[(Benzyloxy)methyl]-6,7-dihydro- 1H,5H-pyrido[3,2,1-ij]-quinazoline-1,3(2H)-dione

6-[(Benzyloxy)methyl]-2-(4-methoxybenzyl)-6,7-dihydro-1 1H,5H-pyrido[3,2,1-ij]quinazoline-1,3(2H)-dione was treated in a similar manner as in Example 41-(4) to give 6-[(benzyloxy)methyl]-6,7-dihydro-1H,5H-pyrido[3,2,1 - ij]quinazoline-1,3(2H)-dione.

$^1$H NMR (DMSO-$d_6$) δ; 11.49 (s, 1H), 7.79 (dd, 1H, J=1.3, 7.9Hz), 7.51 (d, 1H, J=6.4Hz), 7.37-7.24 (m, 5H), 7.16-7.11 (m, 1H), 4.51 (s, 2H), 4.26-4.21 (m, 1H), 3.54-3.39 (m, 3H), 2.99-2.92 (m, 1H), 2.72-2.64 (m, 1H), 2.38-2.28 (m, 1H)

Example 44

Preparation of tert-butyl 1,3-dioxo-2,3,6,7-tetrahydro-1H,5H-pyrido-[3,2,1-ij]quinazoline-6-carbamate:

(1) tert-Butyl 3-[(tert-butoxycarbonyl)amino]-3,4-dihydro-1(2H)-quinolinecarboxylate

To a mixture of 1,2,3,4-tetrahydro-3-quinolinamine (1.92 g, 13.0 mmol) and toluene (10 mL) was added with stirring di-tert-butyl dicarboxynate (6.55 mL, 28.5 mmol) under ice-cooling, and the mixture was stirred at room temperature for 2 hours, and heated under reflux for 4.5 hours. The reaction solution was concentrated, and the resulting residue was purified by silica gel column chromatography ($SiO_2$, hexane : ethyl acetate = 5:1, and then, 3:1) to give tert-butyl 3-[(tert-butoxycarbonyl)amino]-3,4-dihydro-1(2H)-quinolinecarboxylate (4.45 g, yield: 98 %).

$^1$H NMR (DMSO-$d_6$)δ; 7.51 (d, 1H, J=8.1Hz), 7.13-6.95 (m, 4H), 3.80-3.65 (m, 2H), 3.35 (m, 1H), 3.03-2.96 (m, 1H), 2.68-2.59 (m, 1H), 2.50 (s, 9H), 2.48 (s, 9H)

(2) tert-Butyl 8-(aminocarbonyl)-1,2,3,4-tetrahydro-3-quinolyl-carbamate

tert-Butyl 8-(aminocarbonyl)-1,2,3,4-tetrahydro-3-quinolyl-carbamate was obtained in a similar manner as in Example 3-(3) and Example 3-(4).

(3) tert-Butyl 1,3-dioxo-2,3,6,7-tetrahydro-1H,5H-pyrido[3,2,1-ij]-quinazolin-6-ylcarbamate

tert-Butyl 1,3-dioxo-2,3,6,7-tetrahydro- 1H,5H-pyrido[3,2,1-ij]-quinazolin-6-ylcarbamate was obtained in a similar manner as in Example 1-(2).

$^1$H NMR (DMSO-$d_6$) δ; 11.47 (s, 1H), 7.81 (d, 1H, J=6.8Hz), 7.50 (d, 1H, J=6.4Hz), 7.20 (d, 1H, J=7.9Hz), 7.17-7.12 (m, 1H), 4.0-3.91 (m, 2H), 3.78-3.71 (m, 1H), 3.08-3.01 (m, 1H), 2.87-2.80 (m, 1H), 1.37 (s, 9H)

The compound of Example 45 was obtained by deprotecting tert-butyl 1,3-dioxo-2,3,6,7-tetrahydro-1H,5H-pyrido[3,2,1-ij]quinazolin-6-ylcarbamate.

Example 45

6-Amino-6,7-dihydro-1H,5H-pyrido[3,2,1-ij]quinazoline-1,3(2H)-dione hydrochloride

M.p.: >300°C

Example 46

Preparation of 6-hydroxy-5,6-dihydro-1H-pyrrolo[3,2,1-ij]quinazoline-1,3(2H)-dione:

(1) tert-Butyl 3-formyl-1H-indole-1-carboxylate

To a mixture of indole-3-carbaldehyde (5.03 g, 34.7 mmol) and tetrahydrofuran (70 mL) was added with stirring a 60 % sodium hydride (1.52 g, 38 mmol) at room temperature, and the mixture was stirred at 60°C for one hour. The reaction mixture was cooled with ice, and thereto was added dropwise with stirring di-tert-butyl dicarboxynate (8.35 mL,.36.3 mmol) under ice-cooling. After the addition, the ice bath was removed, and the mixture was stirred at room temperature. One hour thereafter, to the mixture was added tetrahydrofuran (50 mL), and the mixture was further stirred overnight. The reaction mixture was slowly poured into cold aqueous ammonium chloride solution, and the mixture was extracted with ethyl acetate. The organic layer was washed with a saturated brine, dried over anhydrous magnesium sulfate, filtered, and concentrated. The resulting residue was roughly purified by silica gel column chromatography ($SiO_2$, hexane : ethyl acetate = 6:1, and then 5:1), and the desired fractions were combined, and concentrated under reduced pressure. To the resulting residue were added hexane and ethyl acetate, and the mixture was stirred. The solid was collected by filtration, and washed with hexane to give tert-butyl 3-formyl-1H-indole-1-carboxyalate (6.36 g, yield: 75 %).

$^1$H NMR ($CDCl_3$) δ; 10.11 (s, 1H), 8.31-8.28 (m, 1H), 8.24 (s, 1H), 8.17-8.14 (m, 1H), 7.45-7.35 (m, 2H), 1.71 (s, 9H)

(2) tert-Butyl 3-(formyloxy)-1 H-indole-1-carboxylate

To a solution of tert-butyl 3-formyl-1H-indole-1-carboxylate (5.38 g, 21.9 mmol) in methylene chloride (150

mL) was added with stirring a 70-75 % m-chloroperbenzoic acid (5.64 g) under ice-cooling. The mixture was stirred for 2 hours under ice-cooling, and then stirred at room temperature overnight. To the reaction mixture were added successively water and a 10 % aqueous sodium sulfite solution, and the mixture was stirred at room temperature for one hour. The organic layer was washed successively with a saturated aqueous sodium hydrogen carbonate solution and a saturated brine, dried over anhydrous magnesium sulfate, filtered, and concentrated. The resulting residue was purified by silica gel column chromatography ($SiO_2$, hexane : ethyl acetate = 10:1, and then 5:1) to give tert-butyl 3-(formyloxy)-1H-indole-1-carboxylate (2.42 g, yield: 42 %).

$^1$H NMR ($CDCl_3$) δ; 8.37 (s, 1H), 8.18 (d, 1H, J=8.3Hz), 7.81 (s, 1H), 7.53 (d, 1H, J=7.7Hz), 7.40-7.34 (m, 1H), 7.30-7.25 (m, 1H), 1.67 (s, 9H)

(3) tert-Butyl 3-oxo-1-indolinecarboxylate

To a mixture of tert-butyl 3-(formyloxy)-1H-indole-1-carboxylate (1.68 g, 6.43 mmol), tetrahydrofuran (10 mL) and methanol (10 mL) was added with stirring a 5 % aqueous potassium carbonate solution (10 mL) under ice-cooling, and the mixture was stirred under ice-cooling for 1.5 hour. The reaction mixture was extracted with ethyl acetate. The organic layer was washed with a saturated brine, dried over anhydrous magnesium sulfate, filtered and concentrated. To the resulting residue were added hexane and ethyl acetate, and the mixture was stirred.
The solid was collected by filtration to give tert-butyl 3-oxo-1-indoline-carboxylate (700.3 mg) as a primary crystal. The filtrate was further concentrated and treated likewise to give tert-butyl 3-oxo-1-indoline-carboxylate (349.5 mg) as a secondary crystal (total yield: 70 %).

$^1$H NMR ($CDCl_3$) δ; 8.23 (br, 1H), 7.73-7.70 (m, 1H), 7.65-7.60 (m, 1H), 7.16-7.11 (m, 1H), 4.22 (s, 2H), 1.59 (s, 9H)

(4) tert-Butyl 3-hydroxy-1-indolinecarboxylate

To a mixture of tert-butyl 3-oxo-1-indolinecarboxylate (582.7 mg, 2.50 mmol), tetrahydrofuran (5 mL) and methanol (0.2 mL) was added with stirring sodium borohydride (28.4 mg, 0.751 mmol) under ice-cooling, and the mixture was stirred under ice-cooling for one hour, and further stirred at room temperature overnight. The reaction mixture was cooled with ice, and thereto was added sodium borohydride (10.1 mg, 0.267 mmol), and the mixture was further stirred under ice-cooling for additional one hour. Water was added to the reaction mixture, and the mixture was stirred for 30 minutes, and extracted with ethyl acetate. The organic layer was washed with a saturated brine and concentrated to give tert-butyl 3-hydroxy-1-indolinecarboxylate (594.8 mg) as a crude product.

(5) 6-Hydroxy-5,6-dihydro- 1H-pyrrolo[3,2,1-ij]quinazoline-1,3(2H)-dione

6-Hydroxy-5,6-dihydro- 1H-pyrrolo(3,2,1-ij]quinazoline-1,3(2H)-dione was obtained in a similar manner as in Example 3.

M.p.: 260-266°C (decomposed)

<u>Example 47</u>

Preparation of 5,6,7,8-tetrahydro-1H-[1,4]diazepino[6,7,1-ij]quinazoline-1,3(2H)-dione hydrochloride:

(1) 4-Trimethyl-2,3,4,5-tetrahydro-1H-1,4-benzoazepine

To a mixture of 2,3,4,5-tetrahydro-1H-1,4-benzodiazepine dihydrochloride (1.61 g), potassium carbonate (3.20 g, 23.2 mmol) and dimethylformamide (8 mL) was added with stirring trityl chloride (1.62 g, 5.81 mmol) at room temperature, and the mixture was stirred at room temperature overnight. Water was added to the reaction mixture, and the mixture was extracted with diethyl ether. The organic layer was washed with a saturated brine, dried over anhydrous sodium sulfate, filtered, and concentrated. The residue was purified by silica gel column chromatography ($SiO_2$, hexane : ethyl acetate = 10:1, and then 5:1) to give crude 4-trityl-2,3,4,5-tetrahydro-1H-1,4-benzodiazepine (1.91 g).

(2) tert-Butyl 4-trityl-2,3,4,5-tetrahydro-1H-1,4-benzodiazepine-1-carboxylate

A mixture of crude 4-trityl-2,3,4,5-tetrahydro-1H-1,4-benzodiazepine(1.91 g), di-tert-butyl dicarbonate (1.40 mL, 6.09 mmol) and toluene (5 mL) was heated under reflux. Three hours thereafter, to the mixture was added di-tert-butyl dicarboxynate (0.50 mL, 2.2 mmol), and the mixture was heated under reflux for one hour. The reaction mixture was cooled with ice, and thereto was added a 29 % aqueous ammonia, and the mixture was stirred at room temperature

overnight. Water was added to the reaction mixture, and the mixture was extracted with diethyl ether. The organic layer was washed with a saturated brine, dried over anhydrous sodium sulfate, filtered, and concentrated. The resulting residue was purifed by silica gel column chromatography ($SiO_2$, hexane : ethyl acetate = 12:1, and then 10:1) to give tert-butyl 4-trityl-2,3,4,5-tetrahydro-1H-1,4-benzodiazepine-1-carboxylate (1.38 g).

(3) 7-Trityl-5,6,7,8-tetrahydro-1H-[1,4]diazepino[6,7,1-ij]-quinazoline-1,3(2H)-dione

7-Trityl-5,6,7,8-tetrahydro- 1H-[1,4]diazepino[6,7,1-ij]-quinazoline-1,3(2H)-dione was obtained in a similar manner as in Example 3.

$^1$H NMR (DMSO-$d_6$) δ; 11.60 (s, 1H), 7.92 (dd, 1H, J=1.6, 7.6Hz), 7.32-7.13 (m, 17H), 4.01-3.91 (br, 2H), 3.73 (s, 2H), 2.79 (br, 2H)

(4) 5,6,7,8-Tetrahydro-1H-[1,4]diazepino[6,7,l-ij]quinazoline-1,3(2H)-dione hydrochloride

A mixture of 7-trityl-5,6,7,8-tetrahydro-1H-[1,4]diazepino[6,7,1-ij]quinazoline-1,3(2H)-dione (91.4 mg, 0.199 mmol), dioxane (2 mL) and a 4N solution of hydrogen chloride in 1,4-dioxane (2 mL, 8.0 mmol) was stirred at room temperature for 3 hours. To the reaction solution was added ether, and the mixture was stirred. The solid was collected by filtration, and washed with diethyl ether to give 5,6,7,8-tetrahydro-1H-[1,4]diazepino[6,7,1-ij]quinazoline-1,3(2H)-dione hydrochloride (50.6 mg, quantitatively).

$^1$H NMR (DMSO-$d_6$)δ;11.71 (s, 1H), 9.88 (br, 2H), 7.98 (dd, 1H, J=1.5, 7.8Hz), 7.69 (dd, 1H, J=1.5, 7.5Hz), 7.29-7.24 (m, 1H), 4.64 (s, 2H), 4.47 (t, 2H, J=5.0Hz), 3.48 (br, 2H)

M.p.: >300°C

The compound of Example 48 was obtained in a similar manner as in Example 47.

Example 48

5,6,7,8-Tetrahydro-1H-azepino[3,2,1 -ij]quinazoline-1,3(2H)-dione

M.p.: 192-196°C

Example 49

Preparation of 7- hydroxy-6,7-dihydro-1H,5H-pyrido[3,2,1-ij]-quinazoline-1,3(2H)-dione

To a mixture of 1H,5H-pyrido[3,2,1-ij]quinazoline-1,3,7(2H,6H)-trione (108.1 mg, 0.500 mmol), methanol (20 mL) and tetrahydrofuran (20 mL) was added portionwise sodium borohydride (100.0 mg, 2.635 mmol) over a period of 5 minutes, and the mixture was stirred at room temperature for 20 minutes. The solvent in the reaction mixture was evaporated off, and water was added to the residue, and the mixture was extracted with chloroform. The organic layer was washed with a saturated brine, dried over anhydrous magnesium sulfate, filtered, and concentrated to give 7-hydroxy-6,7-dihydro-1H,5H-pyrido[3,2,1-ij]-quinazoline-1,3(2H)-dione (72.8 mg, yield: 67 %).

$^1$H NMR (DMSO-$d_6$) δ; 11.51 (br s, 1H), 7.87 (dd, 1H, J=1.7, 7.7Hz), 7.72 (dd, 1H, J=1.7, 7.7Hz), 7.21 (t, 1H, J=7.7Hz), 5.62 (d, 1H, J=5.1Hz), 4.77-4.71 (m, 1H), 4.05-3.82 (m, 2H), 2.08-1.87 (m, 2H).

Example 50

1H,5H-Pyrido[3,2,1-ij)quinazoline-1,3(2H)-dione

A mixture of 7-hydroxy-6,7-dihydro-1H,5H-pyrido[3,2,1-ij)-quinazoline-1,3(2H)-dione (20.0 mg, 0.0917 mmol), p-toluenesulfonic acid monohydrate (20.0 mg) and toluene (20 mL) was heated under reflux for one hour. The reaction mixture was cooled to room temperature, and the solvent in the reaction mixture was evaporated off. The residue was dissolved in chloroform, and the resulting solution was washed successively with water and a saturated brine, dried over anhydrous magnesium sulfate, filtered, and concentrated to give 1H,5H-pyrido[3,2,1-ij]quinazoline-1,3(2H)-dione (17.6 mg, yield: 96 %).

$^1$H NMR (DMSO-$d_6$) δ; 11.59 (br s, 1H), 7.68 (dd, 1H, J=1.1, 7.7Hz), 7.36 (dd, 1H, J=1.1, 7.7Hz), 7.09 (t, 1H, J=7.7Hz), 6.53 (dt, 1H, J=2.9, 10.3Hz), 6.02 (dt, 1H, J=3.7, 10.3Hz), 4.60 (t, 2H, J=2.9Hz)

The compound of Example 51 was obtained in a similar manner as in Example 50.

Example 51

1H-Pyrrolo[3,2,1-ij]quinazoline-1,3(2H)-dione

$^1H$ NMR (DMSO-$d_6$) δ; 11.66 (br s, 1H), 7.96 (d, 1H, J=7.7Hz), 7.87 (d, 1H, J=3.5Hz), 7.79 (d, 1H, J=7.7Hz), 7.42 (t, 1H, J=7.7Hz), 6.93 (d, 1H, J=3.5Hz)

Example 52

7-Chloro-1,3-dioxo-2,3-dihydro-1H,5H-pyrido[3,2,1-ij]quinazoline-6-carbaldehyde

To dimethylformamide (2 mL) was added with stirring phosphorus oxytrichloride (2.21 g, 14.4 mmol) under ice-cooling, and after the ice bath was removed, the mixture was stirred at room temperature for one hour. The Vilsmeier reagent thus obtained was added dropwise to a suspension of 1H,5H-pyrido[3,2,1-ij]quinazoline-1,3,7(2H,6H)-trione (1.30 g, 6.01 mmol) in dimethylformamide (8 mL). After the addition, the mixture was stirred at 80°C for 1.5 hour. To the reaction mixture was added water (100 mL), and the precipitated orange solid was collected by filtration, and washed with water to give 7-chloro-1,3- dioxo-2,3-dihydro- 1H,5H-pyrido[3,2,1-ij]quinazoline-6-carbaldehyde (1.38 g, yield: 87 %).

$^1H$ NMR (DMSO-$d_6$) δ; 11.88 (br s, 1H), 10.17 (s, 1H), 8.08 (d, 1H, J=7.7Hz), 8.03 (d, 1H, J=7.7Hz), 7.32 (t, 1H, J=7.7Hz), 4.67 (s, 2H)

The compound of Example 53 was obtained in a similar manner as in Example 49.

Example 53

7-Chloro-6-(hydroxymethyl)- 1H,5H-pyrido[3,2,1-ij]quinazoline-1,3(2H)-dione

$^1H$ NMR (DMSO-$d_6$) δ; 11.73 (br s, 1H), 7.81 (dd, 1H, J=1.1, 7.7Hz), 7.72 (dd, 1H, J=1.1, 7.7Hz), 7.20 (t, 1H, J=7.7Hz), 5.34 (br s, 1H), 4.74 (s, 2H), 4.35 (s, 2H)

Example 54

7-Chloro-6-(chloromethyl)- 1H,5H-pyrido[3,2,1-ij]quinazoline-1,3(2H)-dione

To a suspension of 7-chloro-6-(hydroxymethyl)-1H,5H-pyrido-[3,2,1-ij]quinazoline-1,3(2H)-dione (100.0 mg, 0.378 mmol) in toluene (20 mL) was added thionyl chloride (258.4 mg, 2.17 mmol), and the mixture was stirred at 80°C for one hour. The reaction mixture was cooled to room temperature, and concentrated under reduced pressure. To the residue was added toluene, and the mixture was further concentrated under reduced pressure to give 7-chloro-6-(chloromethyl)-1H,5H-pyrido[3,2,1-ij]quinazoline-1,3(2H)-dione (107.0 mg, quantitatively).

$^1H$ NMR (DMSO-$d_6$) δ; 11.73 (br s, 1H), 7.83 (dd, 1H, J=1.1, 7.7Hz), 7.75 (dd, 1H, J=1.1, 7.7Hz), 7.19 (t, 1H, J=7.7Hz), 4.72 (s; 2H), 4.54 (s, 2H)

The compound of Example 55 was obtained in a similar manner as in Example 11.

Example 55

7-Chloro-6-[(dimethylamino)methyl]-1 1H,5H-pyrido[3,2,1-ij]quinazoline-1,3(2H)-dione

$^1H$ NMR (DMSO-$d_6$) δ; 11.73 (br s, 1H), 7.84 (dd, 1H, J=1.1, 7.7Hz), 7.77 (dd, 1H, J=1.1, 7.7Hz), 7.23 (t, 1H, J=7.7Hz), 4.68 (s, 2H), 3.31 (s, 2H), 2.20 (s, 6H)

Example 56

1H,5H-Pyrido[3,2,1-ij]quinazoline-1,3,7(2H,6H)-trione 7-oxime

A mixture of 1H,5H-pyrido[3,2,1-ij]quinazoline-1,3,7(2H,6H)-trione (108.1 mg, 0.500 mmol), hydroxylamine hydrochloride (525.0 mg, 7.56 mmol), sodium acetate trihydrate (900.0 mg, 6.609 mmol), ethanol (40 mL) and water (20 mL) was heated under reflux for 6 hours. Ethanol in the reaction mixture was evaporated off, and the precipitated white solid was collected by filtration, and washed with water to give 1H,5H-pyrido[3,2,1-ij]qumazoline-1,3,7(2H,6H)-trione 7-oxime (90.7 mg, yield: 78 %).

$^1H$ NMR ($DMSO-d_6$) δ; 11.61 (br s, 1H), 11.55 (br s, 1H), 8.12 (dd, 1H, J=1.1, 7.7Hz), 7.97 (dd, 1H, J=1.1,.7.7Hz), 7.24 (t, 1H, J=7.7Hz), 3.97 (t, 2H, J=6.6Hz), 2.91 (t, 2H, J=6.6Hz)

The compound of Example 57 was obtained in a similar manner as in Example 56.

Example 57

1H,5H-Pyrido[3,2,1-ij]quinazoline-1,3,7(2H,6H)-trione 7-(O-methyl-oxime)

$^1H$ NMR ($DMSO-d_6$) δ; 11.64 (br s, 1H), 8.13 (dd, 1H, J=1.1, 7.7Hz), 8.00 (dd, 1H, J=1.1, 7.7Hz), 7.25 (t, 1H, J=7.7Hz), 3.97 (t, 2H, J=6.6Hz), 3.96 (s, 3H), 2.92 (t, 2H,J=6.6Hz)

The compound of Example 58 was obtained in a similar manner as in Example 56.

Example 58

1H,5H-Pyrido[3,2,1-ij]quinazoline-1,3,7(2H,6H)-trione 7-(O-benzyloxime)

$^1H$ NMR ($DMSO-d_{6,}$)δ; 11.59 (br s, 1H), 8.70 (dd, 0.5H, J=1.1, 7.7Hz), 8.12 (dd, 0.5H, J=1.1, 7.7Hz), 8.04 (dd, 0.5H, J=1.1, 7.7Hz), 8.00 (dd, 0.5H, J=1.1, 7.7Hz), 7.22-7.45 (m, 6H), 5.23 (s, 1H), 5.20 (s, 1H), 4.04 (t, 1H, J=6.6Hz), 3.97 (t, 1H, J=6.6Hz), 2.97 (t, 1H, J=6.6Hz), 2.69 (t, 1H, J=6.6Hz)

EXPERIMENT

Reagent, apparatus and materials for experiment

- DNA (ultrasonicated) (Nacalai Tesque)
- Nicotinamide adenine dinucleotide (NAD: Nacalai Tesque)
- [$^3H$]NAD ([adenine-2,8-$^3H$]-NAD) (NEN (registered trade name) Life Science Products, Inc. (USA), specific activity: 1402 GBq/mmol)
- PARP (recombinant human PARP, 660 units/mg) (Trevigen, Inc. (USA))
- Benzamide (Wako Pure Chemical Industries, Ltd.)
- 96-well plate (round bottom perfect plate, made of polypropylene) (CORNING Costar (USA))
- glass fiber filter for 96-well plate: printed filter mat B (double thickness, 90×120 mm) (PerkinElmer, Inc. (USA))
- solid scintillant sheet: MeltiLex (registered trade name) A ( 73×109 mm, 4 g/sheet or less) (PerkinElmer, Inc. (USA))
- Sample bag for enclosing filter + scintillant (PerkinElmer, Inc. (USA))

Method of experiment

A buffer (50 mM Tris-HCl (pH8.0)/25 mM $MgCl_2$, aqueous solution) was used in preparation of each solution.

To a 96-well round bottom plate made of polypropylene were added successively a test compound solution (20 μl/well), 1 μM [$^3H$]NAD (specific activity: 7 kBq/ml) containing 10 μg/ml of DNA (30 μl/well), and 4 units/ml (6 μg/ml) PARP solution (50 μl/well), and the plate was placed under room temperature for 1.5 hour for the reaction (final concentrations of each reagent in the reaction solution: DNA: 3 μg/ml; [$^3H$]NAD: 0.3 μM/specifc activity of 2.1 kBq/ml; PARP: 2 units/ml (3 μg/ml)). The reaction was terminated by addition of a 24 mM benzamide (9 μl/well, 2 mM after the addition), and the PARP in the reaction solution was recovered by using a cell harvester (HARVESTER 96 (registered trade name), TOMTEC Inc. (USA)) on the glass fiber filter for plate. Said glass fiber filter had been soaked with 80 % ethanol just prior to the recovary of PARP, and after the recovary of PARP, each well of the plate was washed four times with 80 % ethanol, and the washings were also passed through the glass fiber filter. Said glass fiber filter was dried by heating with a microwave oven for 3 to 4 minutes, and put into a sample bag being sandwiched between 2 solid scintillant sheets, and said sample bag was sealed by heat sealing. The bag was put on a heater at about 50°C to melt the scintillant with heat in order to penetrate it into the filter. The plate was cooled to room temperature, and the [$^3H$] count of poly(ADP-ribose) (cpm) bound to PARP was measured with a counter for plate (1450 MicroBeta (registered trade name) TriLux, PerkinElmer, Inc. (USA)) for 10 minutes per plate.

The inhibitory rate of PARP activity was calculated by the following equation.

$$\text{Inhibitory Rate (\%)} = \left\{1 - \frac{\text{count (cpm) in a well treated with a test compound - background count (cpm)}}{\text{count (cpm) of well not treated with a test compound - background count (cpm)}}\right\} \times 100$$

The inhibitory rate of each test compound was measured in duplicate, and an $IC_{50}$ value thereof was calculated therefrom.

<u>Results</u>

5,6-Dihydro-1H-pyrrolo[3,2,1-ij]quinazoline-1,3(2H)-dione (Compound of Example 1): $IC_{50}$ = 0.14 μM;
6,7-Dihydro- 1H,5H-pyrido[3,2,1-ij]quinazoline-1,3(2H)-dione (Compound of Example 2): $IC_{50}$ = 0.052 μM;
4-(Dimethylamino)-N-[(1,3-dioxo-2,3,6,7-tetrahydro- 1H,5H-pyrido[3,2,1-ij]quinazolin-6-yl)methyl]benzmide (Compound of Example 29): $IC_{50}$ = <0.005 μM;
N-[(1,3-Dioxo-2,3,6,7-tetrahydro-1H,5H-pyrido[3,2,1-ij]quinazolin-6-yl)methyl]-4-piperidinecarboxamide hydrochloride (Compound of Example 39): $IC_{50}$ = 0.0074 μM

INDUSTRIAL APPLICABILITY

The present compounds exhibit a PARP inhibitory activity, by which they can be used in the treatment of diseases caused by accelerated PARP activity such as brain ischemic disorders, neurodegenerative diseases, brain contusion, head injury, spinal injury, diabetes mellitus, ischemic heart diseases, organ damages caused by ischemia or ischemic reperfusion injury, inflammations, inflammatory enteritis, cancers, cachexy, renal damage, osteoporosis, acute pain and chronic pain, septic shock, skeletal muscle degenerative disease, muscular dystrophy, skin aging, aging of immune system, AIDS, alteration of gene expression of senescent cells, etc.

## Claims

**1.** A compound of the formula (1):

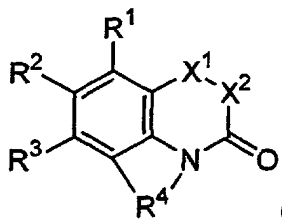

(1)

wherein $-X^1-X^2-$ is a group of the formula: $-C(=O)-N(R^7)-$ or $-C(R^8)=N-$ (in which $R^7$ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated heterocyclic group, or a substituted or unsubstituted acyl group, $R^8$ is a halogen atom or a group of the formula: $-OR^{8a}$, $-NH_2$,$-NHR^{8a}$,$-NR^{8a}R^{8b}$ or $-SR^{8a}$ ($R^{8a}$ and $R^{8b}$ are independently a substituted or unsubstituted alkyl group));

$R^1$, $R^2$ and $R^3$ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated heterocyclic group, a substituted or unsubstituted acyl group, a halogen atom, a nitro group, or a group of the formula: $-OR^{1a}$, $NR^{1a}R^{1b}$ or $-SR^{1a}$ (in which $R^{18}$ and $R^{1b}$ are independently a hydrogen atom, or a substituted or unsubstituted alkyl group);

$R^4$ is a substituted or unsubstituted alkylene group (in which the -$CH_2$- moiety of said alkylene group may optionally be replaced by one or more groups which are the same or different, selected from the group consisting of -O-, -$S(O)_n$-, -N($R^{6a}$)-, -C(=N-O$R^{6b}$)-, -C(=C$R^{6c}R^{6d}$)-, and -C(=O)-, and any combination of two adjacent carbon atoms of said alkylene group may optionally form a double bond or a triple bond, n is an integer of 0, 1 or 2, $R^{6a}$ is a substituted or unsubstituted alkyl group, a substituted or unsubstituted alkenyl group, a substituted or unsubstituted alkynyl group, a substituted or unsubstituted cycloalkyl group, a substituted or unsubstituted cycloalkylalkyl group, a substituted or unsubstituted arylalkyl group, a substituted or unsubstituted aromatic group, a substituted or unsubstituted saturated heterocyclic group, or a substituted or unsubstituted acyl group, $R^{6b}$ is a hydrogen atom, a substituted or unsubstituted alkyl group, or a substituted or unsubstituted arylalkyl group, $R^{6c}$ and $R^{6d}$ are independently a hydrogen atom or a substituted or unsubstituted lower alkyl group),

provided that 1H,5H-pyrido[3,2,1-ij]quinazoline-1,3,7(2H,6H)-trione, 9-methyl-5,6-diphenyl- 1H-pyrrolo[3,2,1-ij]quinazoline-1,3(2H)-dione, and 9-methoxy-5,6-diphenyl-1H-pyrrolo[3,2,1-ij]quinazoline-1,3(2H)-dione are excluded,

or a prodrug thereof, or a pharmaceutically acceptable salt of the same.

**2.** The compound according to claim 1, or a prodrug thereof, or a pharmaceutically acceptable salt of the same, wherein $R^4$ is a substituted or unsubstituted $C_{2-5}$ alkylene group (in which the -$CH_2$-moiety of said alkylene group may optionally be replaced by one or more groups which are the same or different, selected from the group consisting of-O-, -$S(O)_n$-, -N($R^{6a}$)-, -C(=N-O$R^{6b}$)-, -C(=C$R^{6c}R^{6d}$)-, and -C(=O)-, and any combination of two adjacent carbon atoms of said alkylene group may optionally form a double bond or a triple bond, and n, $R^{6a}$, $R^{6b}$, $R^{6c}$, and $R^{6d}$ are as defined in claim 1).

**3.** The compound according to claim 1, or a prodrug thereof, or a pharmaceutically acceptable salt of the same, wherein $R^4$ is a substituted or unsubstituted $C_{2-5}$ alkylene group (in which the -$CH_2$-moiety of said alkylene group may optionally be replaced by one or more groups which are the same or different, selected from the group consisting of -C(=N-O$R^{6b}$)-, -C(C$R^{6c}R^{6d}$)-, and -C(=O)-, and any combination of two adjacent carbon atoms of said alkylene group may optionally form a double bond or a triple bond, and $R^{6b}$, $R^{6c}$, and $R^{6d}$ are as defined in claim 1).

**4.** The compound according to claim 1, or a prodrug thereof, or a pharmaceutically acceptable salt of the same, wherein $R^4$ is a substituted or unsubstituted $C_{2-5}$ alkylene group.

**5.** The compound according to any one of claims 1 to 4, or a prodrug thereof, or a pharmaceutically acceptable salt of the same, wherein $R^4$ has at least one substituent, and at least one of said substituents is a substituted alkyl group of the formula: -$R^{4a}$-$R^{4b}$-$R^{4c}$-$R^{4d}$ (in which $R^{4a}$ is a substituted or unsubstituted alkylene group (among -$CH_2$-groups of said alkylene group, one -$CH_2$- group other than one directly binding to $R^4$ may optionally be replaced by an oxygen atom or a group of the formula: -N$R^{4e}$C(=O)- or -C(=O)N$R^{4e}$ ($R^{4e}$ is a hydrogen atom, a lower alkyl group, or an arylalkyl group)), $R^{4b}$ is a substituted or unsubstituted aromatic group, a cycloalkyl group, or a single bond, $R^{4c}$ is a substituted or unsubstituted alkylene group (one of the -$CH_2$-groups of said alkylene group may optionally be replaced by an oxygen atom) or a single bond, $R^{4d}$ is a hydrogen atom, an amino group or a saturated heterocyclic group containing a nitrogen atom (said amino group or the nitrogen atom of the saturated heterocyclic group containing a nitrogen atom may optionally have one or two lower alkyl substituents or arylalkyl substituents, which are the same or different)).

**6.** A medicament, which comprises the compound as set forth in any one of claims 1 to 5, or a prodrug thereof, or a pharmaceutically acceptable salt of the same.

**7.** A poly(ADP-ribose)polymerase inhibitor, which comprises the compound as set forth in any one of claims 1 to 5, or a prodrug thereof, or a pharmaceutically acceptable salt of the same.

**8.** An agent for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, or skin aging, which comprises the compound as set forth in any one of claims 1 to 5, or a prodrug thereof, or a pharmaceutically acceptable salt of the same.

**9.** A use of the compound as set forth in any one of claims 1 to 5, or a prodrug thereof, or a pharmaceutically acceptable salt of the same, in preparation of a poly(ADP-ribose)polymerase inhibitor.

**10.** A use of the compound as set forth in any one of claims 1 to 5, or a prodrug thereof, or a pharmaceutically acceptable salt of the same, in preparation of an agent for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, or skin aging.

**11.** A method for inhibiting poly(ADP-ribose)polymerase in a patient in need, which comprises administering to said patient the compound as set forth in any one of claims 1 to 5, or a prodrug thereof, or a pharmaceutically acceptable salt of the same.

**12.** A method for treatment of brain ischemic disorders, stroke, aftereffects of stroke, brain edema, neurodegenerative diseases, Parkinson's disease, Alzheimer's disease, Huntington's chorea, brain contusion, head injury, spinal injury, diabetes mellitus, ischemic heart disease, myocardial infarction, myocardial ischemic reperfusion injury, angina pectris, arrhythmia, arthritis, rheumatoid arthritis, inflammatory enteritis, septic shock, cancers, or skin aging, which comprises administering to a patient in need the compound as set forth in any one of claims 1 to 5, or a prodrug thereof, or a pharmaceutically acceptable salt of the same.

## INTERNATIONAL SEARCH REPORT

International application No.
PCT/JP01/03104

A. CLASSIFICATION OF SUBJECT MATTER
Int.Cl[7] C07D487/04, C07D471/04, C07D498/04, C07D513/04, A61K31/519, A61K31/55, A61K31/551, A61K31/5365, A61K31/542, A61P9/10, A61P25/00, A61P25/16, A61P25/28, A61P25/14, A61P3/10, A61P9/06, A61P19/02, A61P29/00, A61P1/04, A61P35/00

According to International Patent Classification (IPC) or to both national classification and IPC

B. FIELDS SEARCHED

Minimum documentation searched (classification system followed by classification symbols)
Int.Cl[7] C07D487/04, C07D471/04, C07D498/04, C07D513/04, A61K31/519, A61K31/55, A61K31/551, A61K31/5365, A61K31/542, A61P9/10, A61P25/00, A61P25/16, A61P25/28, A61P25/14, A61P3/10, A61P9/06, A61P19/02, A61P29/00, A61P1/04, A61P35/00

Documentation searched other than minimum documentation to the extent that such documents are included in the fields searched

Electronic data base consulted during the international search (name of data base and, where practicable, search terms used)
CA(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | GAMA Y. et al., "Synthesis of glucosylamino•••amino acids", 29(9), 1999, pp.1493-1501, table 1 | 1-3 |
| A | BANASIK M. t al., "Specific Inhibitors of • •transferase", Vol.267, No.3, 1992, pp.1569-1575 | 1-10 |
| A | US, 3872119, A (Sandoz-Wander, Inc.), 18 March, 1975 (18.03.75) (Family: none) | 1-10 |
| A | US, 3709887, A (Sandoz-Wander, Inc.), 09 January, 1973 (09.01.73) (Family: none) | 1-10 |
| A | WO, 98/33802, A1 (UNIVERSITY VENTURES LIMITED), 06 August, 1998 (06.08.98) & EP, 966476, A1 & US, 6156739, A | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 June, 2001 (08.06.01) | 19 June, 2001 (19.06.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/03104

**Box I Observations where certain claims were found unsearchable (Continuation of item 1 of first sheet)**

This international search report has not been established in respect of certain claims under Article 17(2)(a) for the following reasons:

1. ☒ Claims Nos.: 11-12
because they relate to subject matter not required to be searched by this Authority, namely:

Claims 11 and 12 involve methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)